# EUROPEAN PATENT APPLICATION

(11) **EP 4 538 366 A1**
(43) Date of publication of application: **16.04.2025**
(21) Application number: 23203204.5
(22) Date of filing: 12.10.2023
(51) Int. Cl.: C12N 5/0783, G01N 33/50

(54) **ENHANCING LINEAGE STABILITY OF THERAPEUTIC REGULATORY T CELL PRODUCTS**

(71) Applicant: Charité - Universitätsmedizin Berlin, 10117 Berlin (DE)
(72) Inventor: SCHMÜCK-HENNERESSE, Michael, 10247 Berlin (DE); WENDERING, Desiree Jacqueline, 14167 Berlin (DE); VOLK, Hans-Dieter, 10117 Berlin (DE)
(74) Representative: Moré, Solveig Helga

(57) **Abstract**

The present invention relates to the field of immunotherapy, in particular, to adoptive T cell therapy with regulatory T cells. The invention provides a composition comprising CD4+CD25+CD127low regulatory T cells having an enhanced lineage stability to inflammatory triggers, as well as methods of preparing such compositions, as well as methods producing regulatory T cell products by expanding such compositions. Regulatory T cell products obtained according to the method of the invention can be used e.g., for treating a subject having or at risk of having an undesired immune response, such as a subject having an autoimmune disease, an allergy or other overwhelming inflammatory diseases, a transplant or a gene therapy recipient.

## Description

The present invention relates to the field of immunotherapy, in particular, to adoptive T cell therapy with regulatory T cells. The invention provides a composition comprising CD4+CD25+CD127low regulatory T cells having an enhanced lineage stability to inflammatory triggers, as well as methods of preparing such compositions, as well as methods producing regulatory T cell products by expanding such compositions. Regulatory T cell products obtained according to the method of the invention can be used e.g., for treating a subject having or at risk of having an undesired immune response, such as a subject having an autoimmune disease, an allergy, sepsis or other inflammatory diseases, a transplant recipient, or a recipient of a gene therapy, e.g., an immunogenic gene therapy.

Regulatory T (T_{REG}) cells are key players in maintaining immune homeostasis, resolution of inflammation and self-tolerance (1). T_{REG} cells employ a variety of molecular mechanisms to control immune responses and therefore have the potential to be used as versatile and adaptable therapeutics for the treatment of autoimmune/inflammatory diseases and unwanted immune responses. In fact, recent early clinical trials of adoptive T_{REG} cell immunotherapy have demonstrated feasibility, tolerability, and potential efficacy for various medical indications including solid organ transplantation (SOT), hematopoietic stem cell transplantation (HSCT), and autoimmunity (2, 3).

T_{REG} cells account for 5% of circulating CD4+ T cells and can - in fixated cells - be identified by the lineage marker Forkhead Box Protein P3 (FoxP3) (4), whose expression intensity has been linked to suppressive function in human T_{REG} populations. Epigenetic analysis reveals demethylation of a special region in the FoxP3 promoter (T_{REG} -specific demethylated region: TSDR) - a proof for Treg lineage classification. T_{REG} cells are incapable of producing IL-2 while expressing high levels of IL-2Rα (CD25), hence, in the absence of IL-2 produced by other cell subtypes, or in the absence of signals from its receptor, there is a decrease in the number and functional activity of T_{REG} cells, leading to inflammation and autoimmunity(5-8). Expression of the IL-7 receptor subunit-a (CD127) is inversely correlated with FoxP3 expression and suppressive function of human T_{REG} cells and can be employed in conjunction with CD25 as a phenotypic marker in purifying T_{REG} cells (CD4+CD25+CD127low T cells). FoxP3 is also transiently upregulated in non-regulatory CD4+CD25- T cells upon activation, however, the strongest indicator of stable T_{REG} cell lineage is demethylation of the T_{REG}-specific demethylated region (TSDR), an evolutionary conserved non-coding regulatory sequence and CpG-rich element within the FoxP3 locus (9). The TSDR is selectively demethylated in permanently differentiated stable FoxP3+ TREG as opposed to effector cells or induced T_{REG} cells (10). However, demethylation of the TSDR cannot be easily determined in living cells.

Adoptive immunotherapy was developed employing effector T cells (T_{EFF}) cells, mainly to treat viral and tumor diseases (11). However, T_{REG} cells are also useful for adoptive immunotherapy if there is an undesired immune response, e.g., in transplant recipients, autoimmunity, and gene therapy.

There are however challenges in the use of T_{REG} cells in adoptive immunotherapy:
Firstly, T_{REG} cells are a small population, making manufacturing of very pure products without significant impurities by non- T_{REG} cells a challenge. Further, under extreme inflammatory conditions, T_{REG} cells may become unstable, lose FoxP3 expression and their immunosuppressive function, and convert to effector T (T_{EFF}) cells, particularly to T_{H17} cells (12).

Although low impurities of non-T_{REG} cells that are common in the recently used first-generation polyclonal T_{REG} products in clinical trials of different international teams were not associated with any safety issue so far (2), limitations of efficacy cannot be ruled out. Most importantly, impurities and instability of T_{REG} phenotype will have a major safety/efficacy issue with the use of next-generation edited T_{REG} products that are in the pipeline.

Advances in chimeric antigen receptor (CAR) development and genome editing over the past years have facilitated genetic optimization of primary T cell therapy in cancer. These technologies are now being applied to improve the specificity and functionality of T_{REG} cells (13). However, impurities of non-T_{REG} effector T cells or instable T_{REG} cells within next-generation T_{REG} products modified by gene transfer or gene editing for redirected specificities (CAR-T_{REG}), enhanced survival (IL-2 overexpression), target tissue homing, or immunosuppressive drug resistance are hardly acceptable because of safety issues.

To enhance safety and efficacy of next-generation T_{REG} products, two options are currently explored:
Stable overexpression of the T_{REG} master regulator FoxP3 by gene transfer, gene editing, or epigenetic editing is a recently developed way to overcome T_{REG} instability and to suppress undesired activity of impurities (14). However, the stabilization to strong inflammatory challenges is limited. Moreover, it requires an additional molecular modification step associated with safety, e.g., genotoxicity and cost issues. Most importantly, the efficacy of any method of targeted FoxP3 overexpression will not achieve expression in >90% of cells, thus, significant numbers of impurities or instable Treg remain.

Similar disadvantages are associated with the suggestion to stabilize the characteristics of regulatory T cells by knocking out receptors for inflammatory cytokines, like IL-6Ry. Said receptor plays an important role in conversion of T_{REG} to non-regulatory T_{H}17 cells.

Alternatively, it has been found that enrichment of naive T_{REG} (CD45RA+CCR7+and/or CD62L+) has several advantages over using full T_{REG} population comprising naive, central memory, effector memory and tiny amounts of T_{EMRA} T_{REG} subsets (15). Thus, naive T_{REG} are almost free of putatively dangerous non- T_{REG} effector cells and are very robust to inflammatory stimuli. However, this approach has a major shortage - the frequency of naive T_{REG} is relatively low, as the majority of T_{REG} cells, even in young adults, express a memory phenotype (15). This results in very low yield of T_{REG} if only naive T_{REG} cells are used as the cell source of manufacturing.

The ultimate objective of T_{REG} cell therapy is to have a robust and cost-effective manufacturing process and to transfer a long-lived T_{REG} cell population with the ability to create an anti-inflammatory milieu while maintaining a stable T_{REG} identity for a favorable safety profile. The present invention thus addresses the problem of providing an advantageous composition of T_{REG} cells that can be used to provide a regulatory T cell product suitable for adoptive immunotherapy, as well as methods of preparing the same.

This problem is solved by the present invention, in particular, by the subject-matter of the claims. The invention provides a composition comprising CD4+CD25+CD127low regulatory T cells, wherein the regulatory T cells comprise:
- CD45RA+CD95- and CCR7+and/or CD62L+ naive regulatory T cells,
- CD45RO+ and CCR7+ and/or CD62L+ central memory regulatory T cells, and
- preferably, CD45RA+ CD95+ and CCR7+ and/or CD62L+ naive-like memory regulatory T cells
wherein the composition does not comprise CD45RO+CCR7-CD62L- effector memory regulatory T cells.

As most CCR7+ cells are also CD62L+, preferably, the composition comprises CD4+CD25+CD127low regulatory T cells, wherein the regulatory T cells comprise:
- CD45RA+CCR7+CD62L+CD95- naive regulatory T cells,
- CD45RO+CCR7+CD62L+ central memory regulatory T cells, and
- preferably, CD45RA+CCR7+CD62L+CD95+ naive-like memory regulatory T cells
wherein the composition does not comprise CD45RO+CCR7-CD62L- effector memory regulatory T cells.

The inventors found that compositions of regulatory T cells comprising the described cell types have an improved lineage stability.

Potential counterparts of the classical differentiation T cell subsets within the T_{REG} population were investigated. To identify the characteristics and subset derivation, T_{REG} cells were polyclonally expanded on CD3 and CD28 coated beads, as used in clinical studies, each T_{REG} cell subset was sorted on the basis of CCR7, CD45RA and CD95, typical markers used for characterisation of the conventional effector T cell subsets, and its fate, stability and function were followed after activation. Each T_{REG} subset was found sensitive to polyclonal activation, and each displays a specific functional profile. T_{REG} compositions of subsets defining T cell differentiation stages were identified and linear coherence of differentiation identified compared to the CD4 non-T_{REG} counterpart from naive > T_{CM} > T_{EM}. T_{REG} cell subsets depict distinct surface marker expression regarding their proliferation status, thymic emigration and prior activation. A T cell receptor (TCR) repertoire analysis reveal disparate TCR repertoire of non-T_{REG} subsets with hints for clonal expansion in effector-memory like T_{REG} subset vs. polyclonal repertoire in the other subsets. Compared to effector memory-like T_{REG} cells, earlier differentiated memory T_{REG} cells show superior autologous responder T cell proliferation suppression and greater expansion. Most importantly, effector memory-like T_{REG} cell forfeit the greatest degree of TSDR demethylation and immunosuppressive function with concomitant production of effector cytokines. Of note, these effector-like T_{REG} cell products have not lost FoxP3 expression, and therefore phenotypically yet resemble thymic-derived T_{REG} cells. Therefore, a composition of T_{REG} cells excluding said effector memory-like T_{REG} cell provides an enhanced lineage stability, i.e., an enhanced stability of regulatory characteristics of the T cells, compared to a population of bulk T_{REG} cells also comprising effector memory-like T_{REG} cells.

The invention thus provides a novel strategy to enrich T_{REG} subsets consisting of naïve, naive-like memory and central-memory phenotype with high and stable immunoregulatory potency and strong robustness to inflammatory stimuli. The inventors could demonstrate that the resulting cell population expresses superior properties compared to whole T_{REG} population and is the ideal cellular source for manufacturing first- and next generation T_{REG} therapy products with high safety and efficacy as well as reasonable yield, and outperforming any method described so far.

In addition to prior art compositions consisting exclusively of naive T_{REG} cells, the composition of the invention additionally comprises central memory T_{REG} cells (T_{REG}CM). The inventors showed that T_{REG}CM exhibit the highest proliferative capacity, independent of the duration and frequency of TCR stimulation, as well as most stable phenotype after activation and a high degree of TSDR demethylation over time. A regulatory T cell product comprising these cells is thus functionally advantageous in adoptive T cell therapy over a composition comprising only naive T_{REG} cells. It is also easier to produce higher cell numbers, and the inventors found that this comparatively large T_{REG} population can be included without any detriment to safety of the T_{REG} composition. Furthermore, it must be emphasized that patients cannot have more blood drawn to have more starting material and, depending on the therapy, may also have few cells in the peripheral blood with which to start the cell manufacture.

Further, a yet not identified stable and functional population of CD95⁺ (FasR+) cells was described by the inventors among the CCR7⁺CD45RA⁺T_{REG} cell population, which in principle corresponds to T_{SCM}, and which was designated naive-like memory T_{REG} (T_{REG}NLM). This population also has a high lineage stability, and are thus preferably also be comprised in the composition.

The inventors showed that a clear increase in lineage stability and consequently in safety can be obtained by a composition of the invention and regulatory T cell products obtained therefrom, wherein the only T_{REG} cell population that needs to be depleted from the composition is the CD45RO+CCR7-CD62L- effector memory regulatory T cell population. Said population was found to be significantly less stable in its regulatory characteristics than the other T_{REG} subpopulations. Thus, only about 10-20% of the total T_{REG} population are lost compared to high loss rate using currently discussed approaches, like naive T_{REG} enrichment or FoxP3 overexpression.

In the context of the invention, "does not comprise" cells having a specific phenotype means that essentially no cells of this phenotype are present, e.g., less than 5% of the cells of the composition, preferably, less than 3% of the cells of the composition or less than 1% of the cells of the composition have said specific phenotype. Most preferably, no cells of this phenotype are detectable, e.g., by FACS using labelled antibodies to CD45RO, CCR7 and CD62L.

As will be further explained below, said absence of a specific phenotype in the composition of the invention does not mean that said phenotype is also absent or not comprised in T cell products derived from said composition by activation and culture or expansion. The composition of the invention can thus be an intermediate product in the production of a regulatory T cell product. Said regulatory T cell product is then typically administered to a patient.

Reference to specific cell surface proteins (or markers) such as CD45RA, CD45RO, CCR7, CD62L or CD95 in characterization of cell subtypes or phenotypes does not necessarily mean that any or all of these proteins need to be used for selecting or sorting the cells in the preparation of the composition of the invention, but the presence or absence of them can be used to characterize the cell population. The skilled person will be able to detect, using suitable controls, if cells are positive or negative or low or high for a specific marker. Examples of such determinations are found, e.g., in the examples and figures below. Of course, the recited markers can be used for the selection, preferably, as further disclosed herein.

The T_{REG} cells of the invention are T cells, i.e., they are not only CD4+CD25+CD127low, but also typically express a TCR, i.e., they are also CD3+ cells. They can alternatively express a CAR, but, often, engineering of cells will be carried out after purification of the composition of the invention. They are further FoxP3+ cells, which was detected in some experiments shown below. However, as FoxP3 is an intracellular marker, other markers such as CD25 and/or CD127 are used for selection of living T_{REG} cells.

In one preferred embodiment, the composition of the invention does not comprise conventional CD25- CD4+127high T cells, i.e., conventional effector T cells. It may further not comprise other T cells, e.g., CD8 T cells or gamma delta T cells. NK cells and NKT cells are optionally also absent. In one embodiment, the composition of the invention does not comprise any other cell types. However, in culture methods described below, e.g., in the presence of an mTOR-inhibitor such as rapamycin, contaminating cells such as NK or NKT cells die after about a day, so elimination of such cells by active methods is not required, and they can also be present in the composition of the invention.

The invention also provides a method for preparing the composition of the invention, comprising selecting CD4+CD25+CD127low regulatory T cells.

CD4 is a glycoprotein on the surface of a subset of T cells and serves as a co-receptor of the TCR. CD4 is capable of binding to MHC type II. It is expressed by both regulatory and non-regulatory T cells. CD4+ cells can be positively selected, but they are often selected by depletion of CD8+ cells.

CD25 or IL-2 receptor alpha chain is a protein involved in assembly of a high-affinity IL-2 receptor. It is constitutively and highly expressed in regulatory T cells, but can also be found on other cells, e.g., activated B cells, activated conventional (non-regulatory) T cells, NK cells and other innate lymphocytes, thymocytes or macrophages and dendritic cells. Thus, preferably, other markers are additionally analyzed to select for T_{REG} cells.

CD127 or IL-7 receptor subunit alpha is a subunit of the functional IL-7 receptor and Thymic Stromal Lymphopoietin (TSLP) receptors. Downregulation of CD127 distinguishes T_{REG} cells from non-regulatory T cells, facilitating both T_{REG} cell purification and their functional characterization, e.g., in humans, or in human diseases. CD127 low cells can also be CD127- cells.

The material from which the cells are selected typically will be a sample derived from a subject, e.g., a human subject. The subject can be the patient who is later to be treated with a regulatory T cell product of the invention. Alternatively, the subject may be a different subject, optionally, a subject sharing expression of cell surface molecules such as MHC haplotypes with the subject that is to be treated. In this case, the subject can be a relative of the patient who is to be treated later, e.g., a sibling or a child. In a still other embodiment, the subject from which the cells are derived is not a sibling. If MHC haplotypes of donor and recipient are not shared, an immune response may be possible. Appropriate measures can be taken, e.g., if needed, an immunosuppressive treatment. T cells intended for transfer may be genetically modified to avoid a host-versus-graft response, e.g., by downregulating MHC expression or they can be modified to be resistant to immunosuppressive treatment, e.g., as disclosed in (16).

The sample will typically be a blood sample, or a sample derived therefrom, preferably, from a human subject. For example, the cells can be selected from PBMC (peripheral blood mononuclear cells). The sample can also be a tissue sample, e.g., from spleen, thymus, lymph nodes, Peyer's plaques or other immune-associated tissues. The sample can also be derived from a solid tumor or from an inflamed site, e.g., a site affected by an autoimmune response or an allergy. The sample comprises T cells.

In a preferred embodiment, the method of the invention comprises selecting for naive regulatory T cells and central memory regulatory T cells and, preferably, naive-like memory regulatory T cells. These cell populations are incorporated into the composition of the invention. The selection can be effected in distinct steps, e.g., in sequential magnetic sorting. However, selection can also be in the same step as selecting CD4+CD25+CD127low regulatory T cells.

Naïve regulatory T cells and central memory regulatory T cells and, optionally, naive-like memory regulatory T cells can be, e.g., positively selected, i.e., cells are contacted with antibodies or other specific ligands for the respective cell surface marker and then isolated from other cells based on binding to the antibodies or other ligands. Depending on the sorting method employed, antibodies or other ligands may be labelled, e.g., with a fluorescent label. They may also be labelled, e.g., with a magnetic bead, which allows for direct sorting. They may also be bound to a solid support, such as a column or column material, which can also allow for sorting. Antibodies or other ligands may also be indirectly bound to a label, to a bead or other solid support, e.g., through association with a secondary antibody, protein A or protein G or biotin-streptavidin or biotin-avidin binding. Antibodies to different cell surface markers are typically bound to different labels, so that the expression profile of the cells can be assessed in detail. Thus, cells negative for one of the required markers can be excluded. The quantity of expression of markers can also be used for selection, e.g., in FACs-based methods.

In one embodiment, selection can comprise positive selection for expression of CD25 and/or depletion of CD127 cells and/or depletion of CD8+ cells, preferably, all of these. Depleting CD127+ and/or CD8+ cells can be performed at the same time as positive selection for CD25, e.g., in a FACS-based method (such as FACSorting), or in a microfluidic-based sorting method. It can also be performed sequentially, e.g., by any magnetic-based methods. Alternatively, positive selection for expression of CD4 can be used. For example, cells can first be selected for expression of CD4, and then for expression of CD25 and low or absent expression of CD127.

Using these markers, regulatory T cells can be selected from a composition comprising additional cell types.

Additionally, and in any order (e.g., before, afterwards or simultaneously), the specific subtypes of regulatory T cells as defined herein are selected. This can employ, e.g., positive selection.

In a preferred embodiment, in this context, a positive selection includes selection for expression of CCR7. CCR7 is C-C chemokine receptor type 7, also designated CD197. The receptor guides immune cells to immune organs such as lymph nodes or the spleen. It is expressed e.g. on naive and central memory T cells and naive-like memory T cells, but not on effector T cells.

Further, alternatively or additionally, the positive selection includes selection for expression of CD62L. The T_{REG} cell populations expressing CCR7 and CD62L strongly overlap, so that it is sufficient to select for one of these markers.

CD62L or L-selectin is a cell adhesion protein that can bind to sialylated carbohydrate groups containing a Sialyl LewisX (sLeX) determinant. It facilitates leukocyte-endothelial cell adhesion events and is essential for homing into secondary lymphoid organs. It is also expressed e.g. on naïve and central memory T cells and naive-like memory T cells, but not on different kinds of effector T cells.

As shown by the inventors, these markers also apply for regulatory T cells, and they can thus be used to eliminate CD45RO+CCR7-CD62L- effector memory regulatory T cells.

CD45RA is an isoform of CD45 (protein tyrosine phosphatase, receptor type C (PTPRC) or L-CA) expressed by naive and terminally differentiated T cells, while CD45RO is the isoform expressed by memory T cells. CD45RO or CD45RA expression can also be assessed in the context of the method of the invention, but it is preferably not used for selection, as elimination of CD45RO+ cells or positive selection of CD45RA+ cells would not only exclude effector memory regulatory T cells, but also CD45RO+CCR7+CD62L+ central memory regulatory T cells. The inventors have shown that it is beneficial to keep said cell type in regulatory T cell products.

CD45RA+CD95+ and CCR7+ and/or CD62L+ naive-like memory regulatory T cells (preferably, CD45RA+CCR7+CD62L+CD95+) are preferably also comprised in the composition of the invention. CD95 or FasR or APO-1 belongs to the TNF receptor family, and after binding its ligand Fas, apoptosis of the cells expressing CD95 can be induced. CD95 is expressed, e.g., by activated T cells, in particular, by T_{SCM} in the CD25-CD4+ T cell population and in the T_{REG} population naive-like memory T_{REG} (T_{REG}NLM) found by the inventors. These cells are expected to have a high capacity for self-renewal, and, as their good stability of regulatory characteristics has been shown herein, it is therefore advantageous to include them in the T cell compositions of the invention.

Thus, T_{REG} cells can be obtained, e.g., by sorting cells (e.g., peripheral blood cells or spleen cells) for their expression of CD4, CD25, CD127, CD62L and/or CCR7.

Cell sorting can be carried out in several steps, e.g., two or three steps, or, preferably, in one step. Generally, selection can comprise a) contacting cells with antibodies to a cell surface molecule that is to be selected for and b) cell sorting. Cell sorting can be carried out by a method such as fluorescence-activated cell-sorting (FACS), magnetic-activated cell sorting (e.g. MACS, including sorting with magnetic dynabeads or MACS beads) and microfluidics. Typically, as known in the art, at least in FACS, cells are also sorted for forward and sideward scatter to focus on living cells.

Cell sorting can be positive selection, e.g., cells expressing CD4, CD25, CCR7 and/or CD62L can be positively selected, e.g., by a cell sorting method. Alternatively or additionally, cells can also be negatively selected, e.g., CD127 high cells and/or CD8+ cells can be excluded. CD45RO+CCR7-CD62L- effector memory regulatory T cells can also be negatively selected, e.g., sequestered based on binding of an antibody or ligand to a specific cell surface antigen, e.g., linked to a magnetic bead or another selectable label. The inventors have for example found that effector memory regulatory T cells express CD25 at particularly high quantities.

Selection of the composition of the invention can comprise a mixture of positive and negative selection steps. Cells can also be depleted by negative selection. In addition to sorting methods such as FACS, for example, depletion can comprise binding antibodies to the target molecule on the cell, and contacting the cells with beads capable of binding to said antibodies e.g., through secondary antibodies. Methods such as antibody-dependent cytotoxicity can also be used for negative depletion. In one embodiment, effector memory regulatory T cells are negatively selected, e.g., from a population of regulatory T cells. Other steps of cell selection can also be included in the method of the invention, e.g., T cells can be selected from PBMC by adhesion to nylon wool.

In a preferred embodiment, GMP grade cell sorters, e.g., magnetic cell sorters such as CliniMacs^{®} from Miltenyi Biotec, can be employed to prepare a composition of the invention, in particular, if large scale preparations are desired. FACS or microfluidics-based cell sorters have the advantage that a one-step selection process is possible by combining different markers and selection principles (positive/negative at one step).

To provide the composition of the invention, a simple one-step procedure to enrich the recited three T_{REG} subsets but deplete effector T_{REG} (and non-T_{REG} impurities) by any sorting technology (MACS, FACS, microfluidic etc.) can be used, using antibodies targeting specific surface molecules, such as anti-CD62L and/or anti-CCR7.

Preferably, a stepwise method is used to get maximal purity and yield, e.g.,
- magnetic sorting (e.g., CliniMacs^{®}, Miltenyi Biotech) first negative enrichment for CD4+ (i.e., depletion of CD8+ cells) and then positive enrichment for CD25+, or
- negative enrichment CD4+ cells (i.e., depletion of CD8+ cells) by magnetic sorting (e.g., CliniMacs^{®}), and then microfluidic cell sorting (e.g., with Tyto ^{®}, Miltenyi Biotech) for CD25+ CD127- cells.

Enrichment of CD26L+ and/or CCR7+ (one is sufficient) can be added in both options.

The present invention also provides a method for preparing a regulatory T cell product, comprising carrying out the method for preparing the composition of the invention, as described herein, and activating and culturing the composition prepared by said method. Activating and culturing typically comprises expanding the cells of the composition. Expansion, i.e., an increase of cell numbers typically starts about 4-5 days after activation.

Alternatively, the expansion may also take place in vivo, after administration to a subject, typically, a patient.

The activation and culture, preferably expansion of the regulatory T cells of the composition invention may take place, e.g., for 1 day to 5 weeks, 1 week to 4 weeks or, preferably 2-3 weeks.

Activation may comprise stimulation of cells with anti-CD3 and anti-CD28, e.g., on a solid support such as beads, or on a matrix, preferably, in the presence of suitable cytokines, such as IL-2. In the presence of an mTOR-inhibitor, e.g., rapamycin, purity of the obtained regulatory T cells can be further enhanced. Activation with antigen-presenting cells, or stimulation with MCH-peptide-complexes and anti-CD28 may alternatively be employed. Preferably, the activation does not require antigen-specific cells if the T cell product is to be used for clinical purposes. In one embodiment, the composition of regulatory T cells of the invention is stimulated with anti-CD3 and anti-CD28 beads in the presence of IL-2 and rapamycin. Cells can be restimulated with such beads after some days, e.g., after about a week, which can be repeated every week. The bead to cell ratio can be, e.g., 5:1 to 1:2, preferably, 4:1 to 1:1. For example, a bead to cell ratio of more than 1:1, e.g., 4:1 can be used for the first round of stimulation, and a bead to cell ratio of 1:1 can be used for the following two stimulations on day 7 and day 14.

The methods of the invention may further comprise engineering the cells, in particular, the cells of the inventive composition, e.g., before expansion. Engineering may also take place during expansion or after expansion. Typically, engineering is performed after purification of the composition of the invention.

For example, the cells may be engineered, alternatively, or cumulatively
a) to express a at least one transgene selected from the group comprising a chimeric antigen receptor (CAR) and a transgenic T cell receptor (TCR). This defines the target antigen of the regulatory T cells. The target antigen may, e.g., be a target antigen involved in an immune response to a transplant, e.g., HLA, or a target antigen associated with an autoimmune disease or an allergy, e.g., due to a cross-reactivity. Exemplary target antigens are HLA-A2, autoantigens like insulin, immune-activated tissue antigens like MIG-1a or citrulli-nated proteins;
b) to have improved cell survival, e.g., by overexpression of growth factors like IL-2 or FoxP3 overexpression;
c) to be resistant to an immunosuppressive drug. One option towards this end is an immunophilin knockout alone or in combination with steroid receptor knockout, e.g., as disclosed in (16). The immunophilin may be FK506-binding protein 12 (FKBP12) or cyclophilin A. This may make the cells resistant to the action of the immunosuppressant Tacrolimus and/or cyclosporine A. Immunophilin knockout can be combined with knockout of the nuclear receptor subfamily 3 group C member 1 (NR3C1; the gene encoding for the glucocorticoid receptor). The cell is then resistant to the immunosuppressants Tacrolimus and/or cyclosporine A as well as, optionally, the immunosuppressive corticosteroids (cortisol, predniso-lone, methylprednisolon, dexamethasone).

The method of the invention may further comprise formulating a regulatory T cell product for administration to a subject. For example, additional e.g., non-cellular impurities may be eliminated, and/or a solvent or buffer suitable for administration to a human subject admixed with the cells. The regulatory T cells product may further optionally comprise excipients that may help with storage of the cells. The concentration of cells may be adapted, e.g., the cells may be concentrated or diluted to obtain a desired concentration.

The present invention also provides a composition obtainable from the method of the invention, in particular, a regulatory T cell product. The cells in said regulatory T cell product obtainable from a method of the invention are preferably expanded from the composition of the invention. As explained above, they do not necessarily have the same cell surface proteins as the cells of the composition of the invention.

The composition of the invention may be a pharmaceutical regulatory T cell product, i.e., a regulatory T cell product intended for medical use and administration to a non-human or preferably human subject. The composition typically also comprises a solvent, e.g., water, or a buffer and/or excipient.

Said pharmaceutical regulatory T cell product may be for use in treating a subject having or at risk of having an undesired immune response, such as a subject having an autoimmune disease, an allergy or sepsis or a transplant recipient. The invention also discloses a method of treating a subject having or at risk of having an undesired immune response, such as a subject having an autoimmune disease, an allergy or sepsis or a transplant recipient or a gene therapy recipient, comprising administering the pharmaceutical regulatory T cell product (or an effective amount thereof) to the subject.

The regulatory T cell product of the invention may, optionally, comprise only regulatory T cells.

Alternatively, after purification and, optionally, engineering of the regulatory T cell composition of the invention, the composition can be mixed with a non-regulatory CD25- T cell population and/or administered to a subject simultaneously or one after the other. This can be of interest, e.g., in the context of transplantation, where regulatory T cells may serve to suppress rejection of the transplant, while conventional non-regulatory T cells (e.g., CD25- CD4+ and/or CD8+ T cells), gamma delta T cells, NK cells and/or NK T-cells, optionally, expressing a chimeric antigen receptor or a transgenic TCR, may serve to counter an infection, e.g. with a virus, or to control a tumor.The cited literature is herewith fully incorporated by reference.

The invention is further illustrated and exemplified by the following examples and figures. These are not to be considered as limiting the invention.

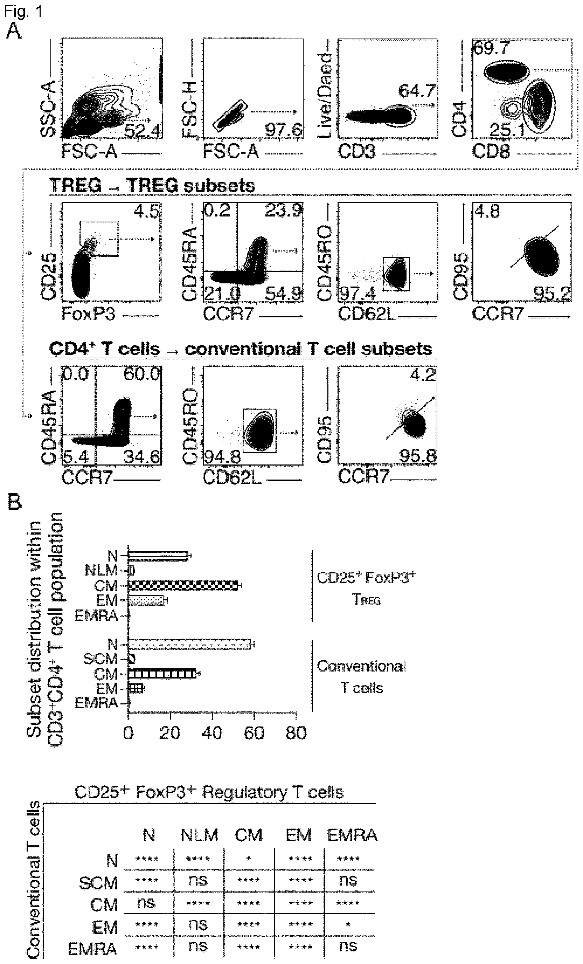

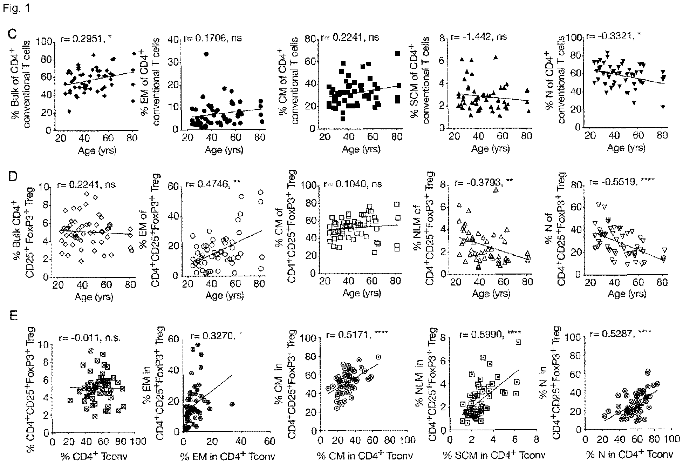

### Figure 1: Definition of naive and memory T_{REG} subsets and comparison to their conventional counterpart

For the *ex vivo* investigation of the phenotype of T_{CONV} and T_{REG}, an extensive flow cytometry panel has been established, defining subsets within both T cell lineages. Freshly isolated PBMCs of 53 healthy donors were labelled with a selection of monoclonal antibodies for flow cytometric analysis. For all flow cytometry-based analyses, the gating strategy commenced with lymphocyte discrimination, doublet exclusion, followed by the selection of living CD3⁺CD4⁺ T cells. From CD4⁺ T cells, T_{REG} were gated based on their high expression of CD25 and FoxP3. Further, T_{REG} memory subsets (T_{REG}CM, T_{REG}EM, T_{REG}TEMRA) were defined according to CD45RA and CCR7 expression. After stringent elimination of any memory T cells by excluding CD45RO⁺CD62L⁻ T cells, T_{REG}NLM and T_{REG}N were defined based on their differential expression of CD95 and CCR7.

Conventional CD4⁺ T cell memory subsets (TCM, TEM, TEMRA) were defined according to their CD45RA and CCR7 expression. Once having excluded CD45RO⁺CD62L⁻ memory T cells, the subsets TSCM and TN were defined by means of their differential expression of CD95 and CCR7.

### Composition of subsets defining T cell differentiation stages suggests coherence in memory formation between bulk CD4⁺ T cells and T_{REG} cells

All conventional memory subsets could be retrieved in the regulatory compartment. However, the central and effector memory population within the bulk CD4⁺ T cells (32.2% and 7.1% respectively) compares to only half the frequency of T_{REG}CM (52.0%) and T_{REG}EM (16.8%). Thereby, the majority of T_{REG} exhibit a central memory phenotype. Further, TN of bulk CD4⁺ T cells (58.3%) are twice as frequent compared to the naive cells within the T_{REG} population. Noting that naive, central and effector memory composition differs strongly between bulk CD4⁺ T cells and T_{REG}, it is intriguing that the frequencies of T_{SCM} within the bulk CD4⁺ T cell compartment (2.8%) strongly compare to that of T_{REG}NLM (2.6%) (Fig. 1B).

To investigate whether the heterogenous T_{REG} subset composition changes over the course of a lifetime, total T_{REG} frequencies and T_{REG} subsets were analysed by Pearson correlation. The data reveal no significant correlation between bulk T_{REG} frequencies and age, proposing that T_{REG} frequencies in the periphery remain at a constant level with age. As expected, both terminally differentiated and effector memory T_{REG} frequencies significantly and positively correlate with age, implying a significant increase of late differentiated T_{REG} with age. T_{REG}N and T_{REG}NLM both significantly and negatively correlate with age, suggesting decreased frequencies of both T_{REG} subsets with age (Fig. 1C). Yet, the difference in correlation strength between T_{REG}NLM and T_{REG}N, identified by Pearson correlation coefficients^{®}) of -0.3793 and -0.5519 respectively, proposes a greater decrease in T_{REG}N frequencies with age compared to T_{REG}NLM. Remarkably, frequencies of T_{REG}CM, making up the largest proportion of the T_{REG} compartment (Fig. 1B) shows no significant correlation with age, suggesting a stable T_{REG}CM population over time (Fig. 1C).

Since the plasticity and differentiation potential within the CD4⁺ and CD8⁺ T_{CONV} compartments are well elucidated, potential correlations between T_{REG} subset frequencies and their bulk CD4⁺ T cell counterpart were explored.

Significant positive correlations between all T_{REG} subsets and their respective bulk CD4⁺ T cell populations were identified.

Notably, the strength of correlation between the naive (r=0.5287) and SCM/NLM (r=0.5990) subsets is greater than that of TEMRA (r=0.3804), EM (r=0.3067) and CM (r=0.3402) subsets, implying the strongest relationship between the frequencies of naive and SCM/NLM T_{REG} and their respective CD4⁺ T_{CONV} counterpart. Taken together, these results suggest a coherence in memory formation between T_{REG} and bulk CD4⁺ T cells (Fig. 1E).

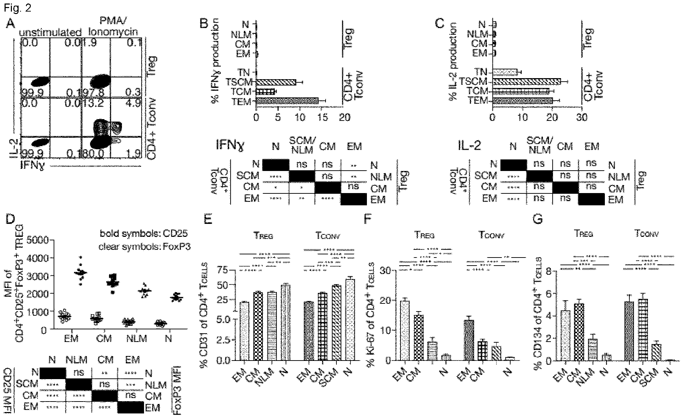

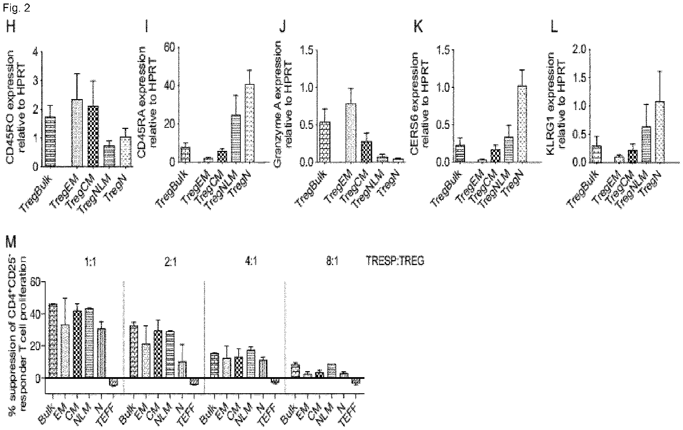

### Figure 2: Phenotypical and functional characterization of memory T cell subsets

Non-production of pro-inflammatory cytokines, including IFNy and IL-2, is highly desired for transfer of stable T_{REG} products used to prevent unwanted immune reactions. Thus, the pro-inflammatory cytokine profile of bulk CD4⁺ T cell and T_{REG} memory subsets was analyzed. In brief, freshly isolated regulatory T cells were polyclonally stimulated with PMA/lonomycin, as well as left untreated and intracellularly stained for IFNγ and IL-2 (Fig. 2A).

The data shown in Figure 2 B and C demonstrate that none of the T_{REG} subsets produce mentionable amount of both IFNγ and IL-2 (each <5%), whilst bulk memory CD4⁺ T cells generate considerable amounts of both cytokines, thereby serving as a positive control for polyclonal stimulation. IFNγ and IL-2 producers are most abundant among TEM, followed by comparable frequencies among TCM and TSCM and least abundant among naive CD4⁺ T cells (Fig. 2 B, C).

The mean fluorescence intensity of the main lineage markers of T_{REG}, namely FoxP3 and CD25 were investigated. Highest intensities of CD25 were observed among T_{REG}EM, significantly decreasing from T_{REG}CM to T_{REG}NLM to T_{REG}N (Fig. 2 D). The same trend was observed for the MFI of FoxP3, however this was not significant for all subsets.

The recent thymic emigrant marker CD31 (PECAM-1) significantly decreased from T_{REG}N to T_{REG}NLM to T_{REG}CM to T_{REG}EM. The same pattern of CD31 expression was detected in the conventional memory subset counterparts (Fig. 2E). The cell division marker Ki-67 showed an entirely contrary expression profile with highest expression by EM > CM > NLN/SCM > N (Fig. 2F). Recent TCR engagement was analyzed by CD134 (OX-40) expression, revealing highest expression among CM and EM, followed by NLM/SCM and very low expression by N (Fig. 2G).

The expression of the differentiation stage-discriminating markers CD45RA and CD45RO were validated on a transcriptional level normalized to the housekeeping gene HPRT.

As expected, CD45RA, expressed on naive CD4⁺ T cells on the protein level, likewise shows greatest mRNA expression in naive T_{REG}. Only slightly lower expression is overserved in T_{REG}NLM compared to T_{REG}N. Very low and neglectable CD45RA expression is found on T_{REG}CM and T_{REG}EM, possibly due to minor T_{REG} subset cross-contamination during the FACS process (Fig. 2I). Messenger-RNA expression of CD45RO, a hallmark surface molecule of memory T cells, indicates to be lowest in both naive and naive memory-like T_{REG} subsets, followed by greater expression by T_{REG}CM and the highest CD45RO expression by T_{REG}EM (Fig. 2H). On a transcriptional level, the inventors were further interested in the expression of ceramide synthase 6 *(CERS6),* which was demonstrated to play a role in the inhibition of T cell activation and differentiation as well as promoting cellular quiescence by regulating intracellular ceramide production. As expected, naive T_{REG} express highest levels of *CERS6*, followed by drastically lower levels expressed by T_{REG}NLM, T_{REG}CM and lowest levels of *CERS6* expressed by T_{REG}EM (Fig. 2K). As thymus-derived activated T_{REG} were reported to express Granzyme A, the transcriptional expression of this cytolytic marker was investigated, and it could thereby be demonstrated that even though normalized mRNA expression levels were generally low, effector T_{REG} present highest expression levels, followed my T_{REG}CM, T_{REG}NLM and lowest expression by T_{REG}N (Fig. 2J).

Since functionality is a defining characteristic of T_{REG} rather than their definition by means of phenotypic markers, none of which are exclusive to the T_{REG} lineage, suppressive capacities of ex *vivo* isolated T_{REG} were studied next. For this, proliferation of polyclonally stimulated autologous responder T cells (T_{RESP}) incubated with different ratios of T_{REG} and was assessed by means of a CFSE dilution-based *in vitro* suppression assay. After 96 hours of co-culture, all T_{REG} subsets contained the capacity to suppress T_{RESP} proliferation. T_{REG}NLM and CM were the most effective inhibitors of T_{RESP} proliferation, especially when co-cultured at ratios 1:1 and 2:1. Naïve T_{REG} show the poorest suppressive capacities (Fig. 2M).

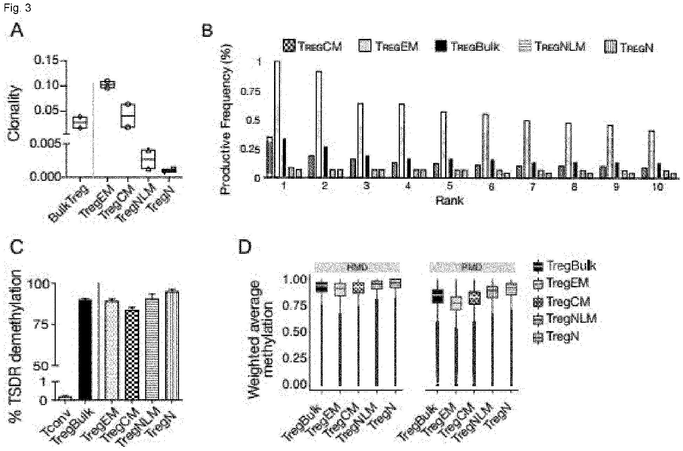

### Figure 3: Clonal diversity and methylation analyses.

TCR repertoire diversity analysis is a common method for analyzing oligoclonal expansion of T cells after cognate antigen encounter offering the opportunity to study different T cell lineages in their process of differentiation thereby helping in elucidating the identity of different T_{REG} subsets and to shed some more light onto the path of T_{REG} differentiation. Of all T_{REG} subsets, T_{REG}EM, being the furthest differentiated T_{REG} subset, demonstrated the greatest clonality (0.1027) and therewith represented the most oligoclonal TCR repertoire (Fig. 3A). Even though the clonality of the two analyzed donors differed from one another, T_{REG}CM revealed to be the population with the next diverse TCR repertoire (0.0041). Particularly remarkable were the clonality measures of the T_{REG}NLM and T_{REG}N subsets. Both populations manifested very low clonalities and therefore presented the two populations with the greatest TCR diversity. However, the T_{REG}NLM subset still had a small, yet considerable larger degree of clonality (0.0027), implying a more oligoclonal TCR repertoire compared to the T_{REG}N population (0.001) (Fig. 3A). Considering the productive frequency of the top 10 clones, it was shown that the top clones entailed higher productive frequencies in the T_{REG}EM subset compared to the top 10 clones of the T_{REG}CM > T_{REG}NLM > T_{REG}N subsets (Fig. 3B).

For the investigation of potential differences in T_{REG}-lineage stability among T_{REG} subsets, the degree of TSDR demethylation of ex vivo T_{REG} sub-populations was analyzed. Controlling for the credibility of the assay, conventional CD4⁺CD25⁻ T cells/NonT_{REG}, shown to have a fully methylated, *i.e.* non-demethylated TSDR, were likewise analyzed. We observed a mean TSDR demethylation of 90.16% within the bulk T_{REG} population. Within the T_{REG} subsets, the central memory population presents with the lowest degree of TSDR demethylation (83.89%), followed by the further differentiated T_{REG}EM subset (89.32%). Remarkably, the earliest differentiated naive T_{REG} subset shows the demethylation of the TSDR (95.03%), closely followed by the T_{REG}NLM subpopulation of 90.75% demethylation. Conventional CD4⁺CD25⁻ T cells/NonT_{REG} display a completely methylated TSDR (0.19% demethylation) (Fig. 3C).

Durek *et al.* reported a progressive loss of DNA methylation from conventional T_{N} > T_{SCM} > T_{CM} > T_{EM}, which was associated with proliferative history (17). To check whether this phenomenon could be retrieved also among the different T_{REG} subsets, a methylation analysis yielding data about highly methylated domains (HMD) and partially methylated domains (PMD) was also carried out. Whereas differences among HMD were less pronounced, a clear increase in weighted average methylation from T_{REG}EM to T_{REG}CM to T_{REG}NLM to T_{REG}N (Fig. 3D) was found, which is completely consistent with the data published for conventional T cell subsets.

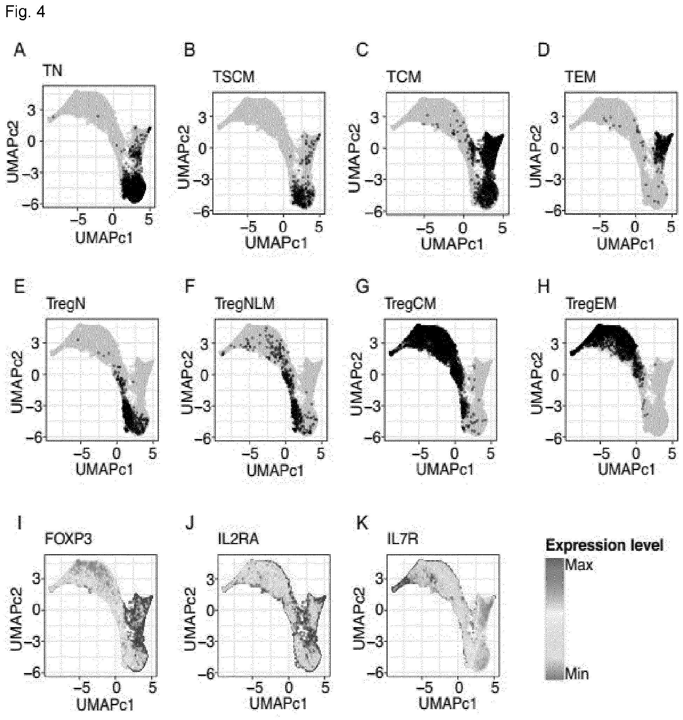

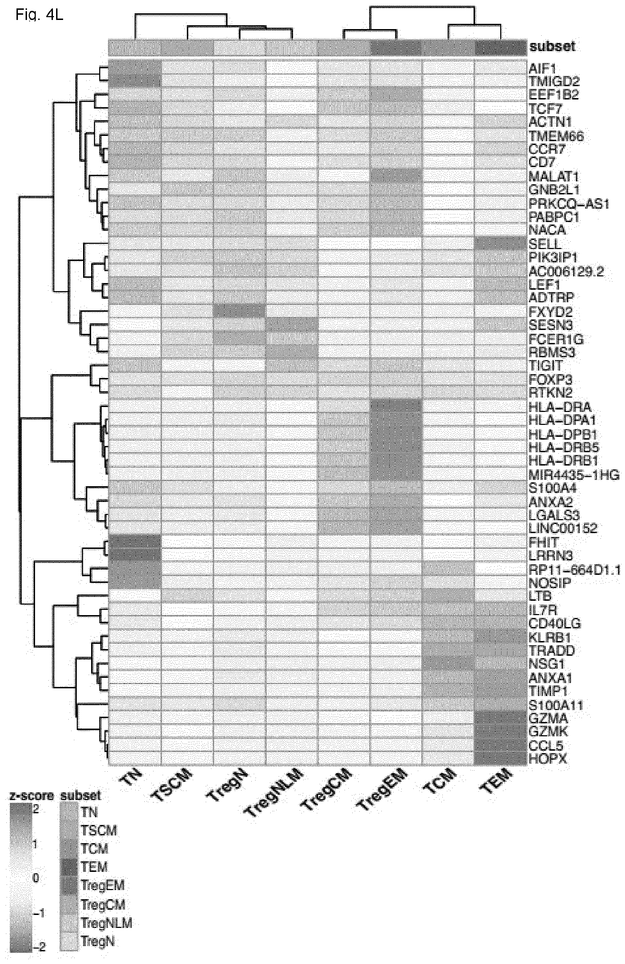

### Figure 4: Single-cell transcriptome and surface epitope analysis (CITE-seq) between CD4⁺ T cells and T_{REG} cells

UMAP representation of FACSorted T_{REG} cells (n=5) and CD4⁺ T cells (n=7) based on top 1K highly variable genes (Fig. 4A-H). Individual plots show subset overlays (black dots) of oligonucleotide-barcoded antibodies-based manual gating. (Fig. 4I-K) Gene expression of canonical T_{REG} markers. Blue to red rainbow color scale denotes low to high expression. (Fig. 4 continuation) Heatmap representation of average expression of top differentially expressed genes between manually gated subsets.

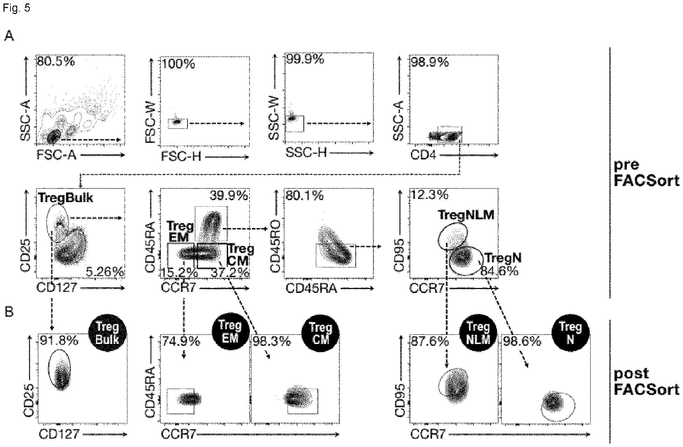

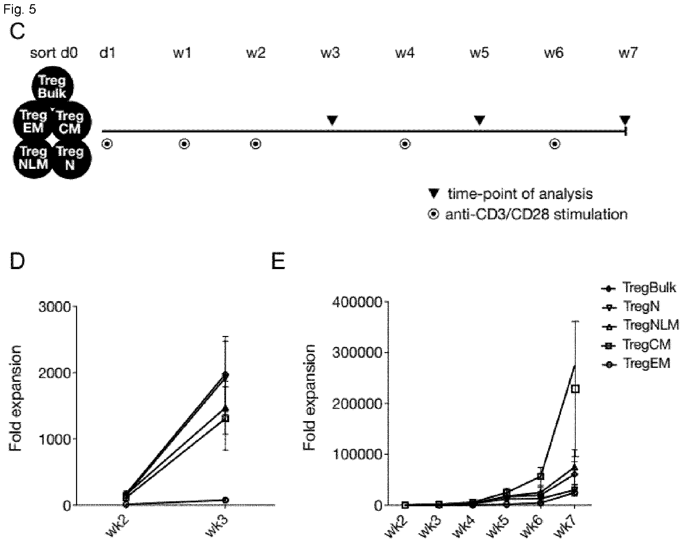

### Figure 5: Sorting and expansion of T_{REG} memory subsets.

To investigate the potential role and relevance of different T_{REG} memory subsets for adoptive T_{REG} therapy, they were sorted using FACS (Fig. 5A, B) and expanded using weekly bead stimulations (Fig. 5C). In the initial phase of expansion and up to week 3, the T_{REG}NLM subset showed greatest expansion, followed by T_{REG}N, T_{REG}CM and T_{REG}EM (Fig. 5D). From week 3 onwards, T_{REG}CM dominated fold expansion over all other T_{REG} subsets and were followed by T_{REG}NLM and bulk T_{REG}, the latter two expanding similarly well (Fig. 5E). Remarkably, lowest expansion was observed in isolated T_{REG}N and T_{REG}EM (Fig. 5E). Between week 4 and 7, all T_{REG} subsets with the exception of T_{REG}EM demonstrated a decrease in fold expansion. Taken together, T_{REG}CM demonstrate the greatest capacity to proliferate.

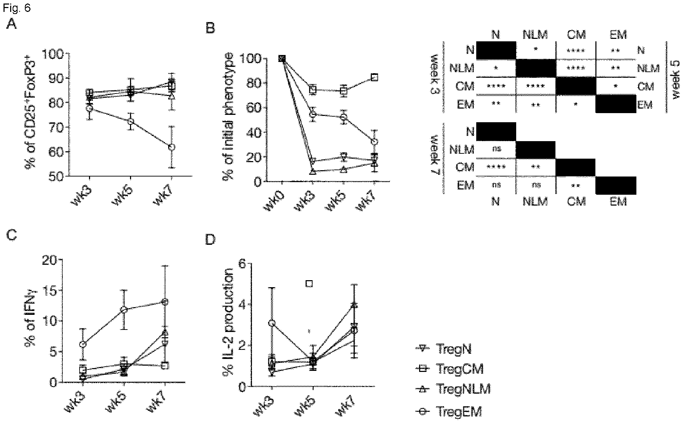

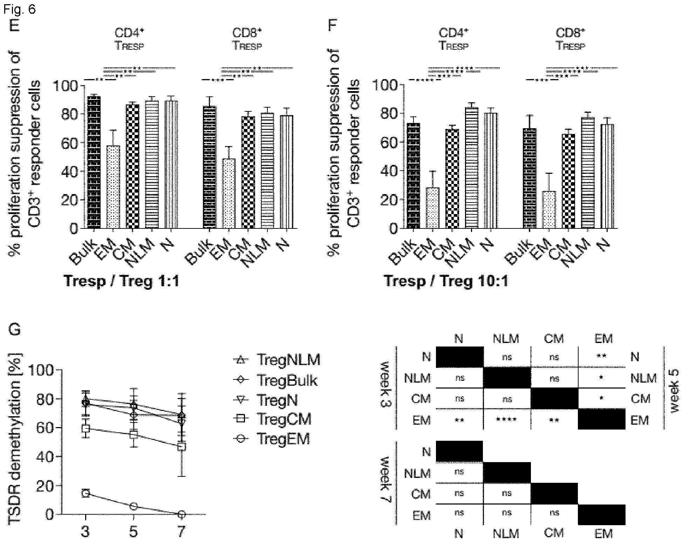

### Figure 6: Characterization of T_{REG} memory subsets after expansion.

With regards to retention of T_{REG} lineage associated proteins CD25 and Foxp3 over a long-term expansion period, all T_{REG} subsets, with the exception of T_{REG}EM, kept their initial CD25⁺FoxP3⁺ expression (Fig. 6A). Approximately 40% of T_{REG}EM lost expression of CD25 and FoxP3 at week 7 (Fig. 6A). To investigate potential differences between T_{REG} subsets, their change of initial naïve/memory phenotype after FACSorting on d0 was monitored over the 7-8 weeks by means of their CD45RA and CCR7 expression. During the initial 3 weeks of expansion, T_{REG}NLM (90%) and T_{REG}N (80%) most drastically lost their initial phenotype of CD45RA⁺ and CCR7⁺ to acquire a central memory-like phenotype of CD45RA⁺ CCR7⁻. 40% of T_{REG}EM also changed their phenotype to an effector memory phenotype of CD45RA⁻ CCR7⁺. Except for T_{REG}EM, all T_{REG} subsets slightly regained their initial phenotype between week 4 and 7 (Fig. 6B).

In addition to the preservation of their phenotype after *in vitro* expansion, another central criterion for the assessment of T_{REG} integrity is the lack of conventional cytokine production (IFNγ and IL-2). After 3 weeks of expansion, both IFNγ and IL-2 producers were negligible (maximum 2.0% IFNγ and 1.21% IL-2 among T_{REG}CM) in all T_{REG} subsets except for T_{REG}EM, which presented an IFNγ and IL-2 production of 6.19% and 3.10% respectively (Fig. 6C, D). IL-2-producers stayed below 5 % in all cases (Fig. 6C). Between week 3 and 7, producers of IFNγ increased, however stayed below 10 % for all memory subsets except for T_{REG}EM (Fig. 6D).

Taken together, IFNγ and IL-2 production by all T_{REG} subsets was negligible until week 3 of expansion, followed by a substantial increase in IFNγ producers among T_{REG}EM, correlating with the loss of their initial phenotype and FoxP3/CD25 expression (Fig., 6A-D).

The capability of the *in vitro* expanded T_{REG} subsets were investigated next. For this, proliferation of freshly isolated autologous CD3⁺ T_{RESP}, which were co-cultured with different ratios of T_{REG}, was assessed *via* an *in vitro* CFSE-dilution assay. After 3 weeks *in vitro* expansion, T_{REG}Bulk, T_{REG}CM, T_{REG}NLM and T_{REG}N co-cultured with autologous T_{RESP} at a 1:1 ratio demonstrate a great capacity to suppress 80-90% of both CD4⁺ and CD8⁺ T_{RESP}. On the other hand, T_{REG}EM only suppressed 57.99% CD4⁺ and 48.86% CD8⁺ T_{RESP} (Fig. 6E). With an increased T_{RESP}:T_{REG} ratio of 10:1, T_{REG}NLM demonstrated greatest suppression of T_{RESP} proliferation over all other T_{REG} subsets, followed by suppression by T_{REG}N and T_{REG}CM and T_{REG}Bulk (Fig. 6F).

To investigate epigenetic stability of the TSDR, expanded T_{REG} subsets were exposed to bisulfite sequencing and qPCR of the TSDR. After the first 3 weeks of expansion, T_{REG}Bulk, T_{REG}NLM and T_{REG}N demonstrated the greatest TSDR demethylation, whereas in comparison, T_{REG}CM presented 20% lower demethylation. Only 13% of the T_{REG}EM TSDR was demethylated at this timepoint (Fig. 6H). In week 5 and 7, the differences of TSDR demethylation between the T_{REG} subsets were comparable with the results of week 3, with less differentiated T_{REG} subsets displaying greatest lineage stability and T_{REG}EM showing a completely methylated TSDR.

In sum, the data demonstrate that indeed the peripheral T_{REG} subset composition of kidney Tx patients shifted to contain a greater proportion of T_{REG}N with the concurrent decrease in T_{REG}CM frequency.

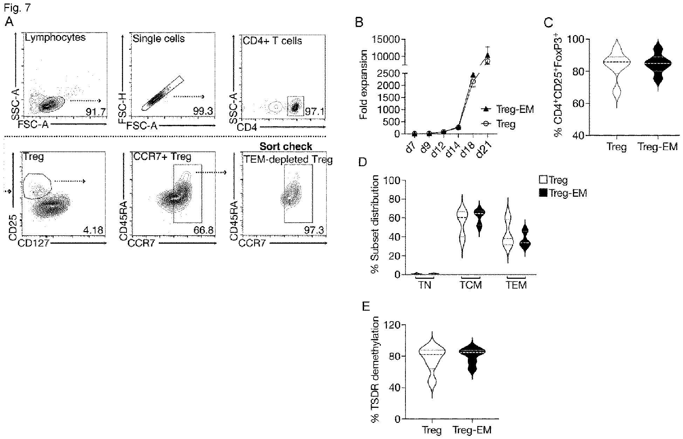

### Figure 7: Depletion of the T_{REG}EM subset population to generate of stable T_{REG} cell products

We investigated the effect of depleting the EM-like T_{REG} subset prior to manufacturing the cell product in order to test the impact of removing this small but instable subset on the functionality of the resulting T_{REG} product. For this purpose, we employed a FACSort to deplete EM cells (CCR7⁻CD45RA⁻) from the initial T_{REG} population, and then compared these cultures with donor-matched bulk T_{REG} cells (Fig. 7A). Over a 21-day period, we observed a modest increase in the expansion of T_{REG}EM-depleted cell product compared to bulk T_{REG} subsets that retained the EM population. The percentage of FoxP3/CD25 positive cells in the final product on day 21 showed no significant difference on average. However, the bulk T_{REG} subset products were more heterogeneous to one another with respect to their final phenotype, frequencies at FoxP3/CD25, and TSDR demethylation (Fig. 7C-E). Depletion of the T_{REG}EM population reduces the overall heterogeneity between T_{REG} cell products from different donors.

Thus, depletion of the T_{REG}EM subset permits the generation of stable T_{REG} cell products in different donors without major impact on yield.

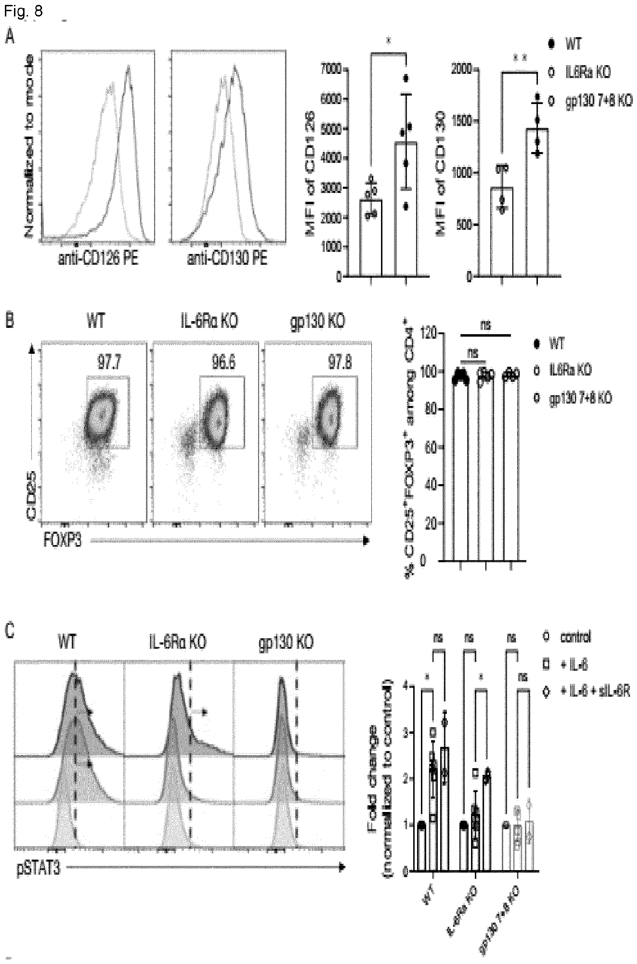

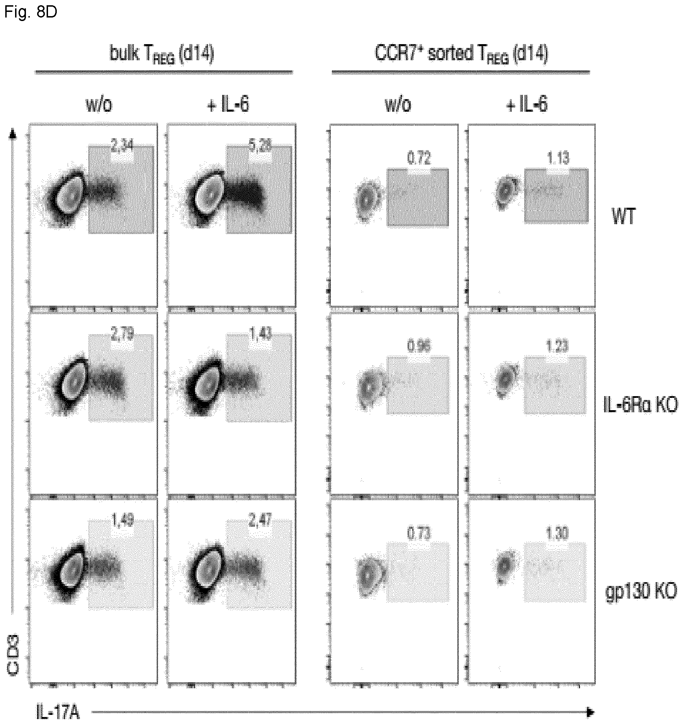

### Figure 8: Enhanced T_{REG} sorting strategy alleviates need for KO of IL-6 pathway to prevent T_{H17} production

We investigated the propensity of T_{REG} cells to dedifferentiate into T_{H17} cells in a strong inflammatory environment using a T_{H17} differentiation protocol based on Koenen et al. (18) To test the impact of IL-6 as a proposed key driver of dedifferentiation, we created a targeted gene knock-out to disrupt the IL-6 receptor complex (IL-6R + gp130) in bulk and CCR7⁺ enriched T_{REG} cells (Fig. 8A-C). Whereas > 5% of wild-type bulk T_{REG} produced IL-17 post IL-6 challenge, IL-6Ra or gp130 knock-out bulk T_{REG} showed a markedly reduced expression of about 1% and 2%, respectively. Remarkably, we found that our sorting strategy for CCR7⁺ T_{REG} (depleting T_{REG}EM) prior to *in vitro* T_{H17} induction culture effectively prevents T_{H17} production in unstimulated and IL-6 stimulated cultures (Fig. 8D).

### Examples

### Results

**Composition of subsets defining T cell differentiation stages suggests coherence in memory formation between bulk CD4⁺ T cells and T_{REG} cells**

To investigate the phenotype of T_{CONV} and T_{REG} *ex vivo,* an extensive flow cytometry panel was utilized, allowing for the identification of subsets within both CD4⁺ T cell lineages. Freshly isolated PBMCs from 53 healthy donors were labeled with specific monoclonal antibodies for flow cytometric analysis (Fig. 1, and methods section). The gating strategy involved lymphocyte discrimination, doublet exclusion, and the selection of living CD3⁺CD4⁺ T cells (Fig. 1). Within the CD4+ T cell population, T_{REG} were identified based on their expression of CD25 and FoxP3 (CD4⁺FoxP3⁺⁺CD25⁺⁺). Conventional memory CD4⁺ T cell T_{CM}, T_{EM} and T_{EMRA} cell subsets were defined according to CD45RA and CCR7 expression, and once having excluded CD45RO⁺CD62L⁻ memory T cells from the CCR7⁺CD45RA⁺ population, the subsets T_{SCM} and T_{N} were defined by means of their differential expression of CD95 and CCR7 (T_{CM}: CCR7⁺CD45RA⁻, T_{EM}: CCR7⁻CD45RA⁻, T_{EMRA}: CCR7⁻CD45RA⁺, T_{N}: CCR7⁺CD45RA⁺CD95⁻, T_{SCM}: CCR7⁺CD45RA⁺CD95⁺). Further, T_{REG} memory subsets (T_{REG}CM, T_{REG}EM, T_{REG}T_{EMRA}) were defined according to CCR7 and CD45RA expression (T_{REG}CM: CCR7⁺CD45RA⁻, T_{REG}EM: CCR7⁻CD45RA⁻, T_{REG}T_{EMRA}: CCR7⁻CD45RA⁺). Likewise, after stringent exclusion of remnant memory T cells by excluding CD45RO⁺CD62L⁻ T cells, memory stem-like CD4⁺ T_{REG} cells, that we nominate naïve-like memory (NLM) and naive T_{REG} cells (T_{REG}N) were delineated based on the differential expression of CD95 and CCR7 (T_{REG}N: CCR7⁺CD45RA⁺CD95⁻, T_{REG}NLM: CCR7⁺CD45RA⁺CD95⁺) (Fig. 1A). All conventional memory and the naive T cell subset could be retrieved in the T_{REG} cell compartment. However, the central and effector memory population within the bulk CD4⁺ T cells (32.2%± and 7.1%±, respectively) compares to only half the frequency of T_{REG}CM (52.0%± ) and T_{REG}EM (16.8%± ). Thereby, the majority of T_{REG} exhibit a central memory phenotype. Of note, T_{N} of bulk CD4⁺ T cells (58.3%±) are twice as frequent compared to the naive cells within the T_{REG} population. Considering the substantial differences in the composition of naive, central, and effector memory cells between bulk CD4⁺ T cells and T_{REG}, il is intriguing that the frequencies of T_{SCM} in the bulk CD4⁺ T cell compartment (2.8%± ) closely resemble those of T_{REG}NLM (2.6%± ) (Fig. 1B).

To investigate whether the heterogenous T_{REG} cell subset composition changes over the course of a lifetime toward a higher abundance of memory cells at the expense of the naive compartment, analogous to the bulk CD4⁺ T cell compartment, the total T_{REG} cell frequencies and T_{REG} subsets were analysed by Pearson correlation as a function of chronological age (Fig. 1C-E). The data reveal no significant correlation between bulk T_{REG} frequencies and age, proposing that T_{REG} cell frequencies in the periphery remain at a constant level with age. Both terminally differentiated and effector memory T_{REG} frequencies significantly and positively correlate with age, implying a significant increase of late differentiated T_{REG} cells with age. Both, T_{REG}N and T_{REG}NLM cells correlate significantly and negatively with age, suggesting that the frequency of either T_{REG} cell subsets decreases with age (Fig. 1C). Yet, the difference in correlation strength between T_{REG}NLM and T_{REG}N, as determined by Pearson correlation coefficient (r) of -0.3793 and -0.5519 respectively, suggests a greater decrease in T_{REG}N frequencies with age compared with T_{REG}NLM. Remarkably, the frequencies of T_{REG}CM, which constitute the largest proportion of the T_{REG} compartment (Fig. 1B), shows no significant correlation with age, suggesting a stable T_{REG}CM population over time (Fig. 1C).

Since the plasticity and differentiation potential within the CD4⁺ and CD8⁺ T_{CONV} cell compartments are well elucidated, we investigated possible correlations between the frequencies of T_{REG} cell subset and their CD4⁺ T cell counterpart. We found significant positive correlations between all T_{REG} cell subsets and their respective bulk CD4⁺ T cell populations. Of note, the strength of correlation between the naive (r=0.5287) and SCM/NLM (r=0.5990) subsets is greater than that of T_{EMRA} (r=0.3804), EM (r=0.3067) and CM (r=0.3402) subsets, implying that the strongest relationship is between the frequencies of naive and SCM/NLM T_{REG} and their respective CD4⁺ T_{CONV} counterpart. Taken together, these results suggest coherence in memory formation between T_{REG} and bulk CD4⁺ T cells (Fig. 1E), as the memory-like T_{REG} cell subsets can be designated in comparison to their conventional CD4⁺ T cell counterpart.

**Phenotypical and functional ex *vivo* characterization of CD4⁺ T_{CONV} and T_{REG} cell subsets.**

Typically, T_{REG} cells do not produce pro-inflammatory cytokines such as IFNγ and IL-2, and the lack of production in response to TCR-specific stimulus is characteristic. Thus, the pro-inflammatory cytokine profile was analyzed after intracellular detection of IFNγ and IL-2 from freshly isolated bulk CD4⁺ T cell and T_{REG} cell subsets upon polyclonal stimulation with PMA/ionomycin compared with unstimulated cells (Fig. 2A). Neither T_{REG} subsets produce notable amounts of both IFNγ and IL-2 (each <5%), whilst bulk memory CD4⁺ T cells produce substantial amounts of both cytokines, thereby serve as a positive control for polyclonal stimulation (Fig. 2 B, C). IFNγ and IL-2 producers are most abundant among conventional CD4⁺ T_{EM} cells, followed by comparable frequencies among T_{CM} and T_{SCM} cells, and least abundant among naive CD4⁺ T cells (Fig. 2 B, C).

For a comprehensive phenotypical characterization of T_{REG} cell subsets, we assessed the mean fluorescence intensity (MFI) of the T_{REG} lineage determining markers FoxP3 and CD25. Highest intensities of CD25 were observed among T_{REG}EM, which decreased significantly from T_{REG}CM to T_{REG}NLM to T_{REG}N (Fig. 2D). No significant differences were found in any subsets regarding the MFI of FoxP3, indicating a similar trend. CD31 (PECAM-1) as a marker for recent thymic emigrants decreased significantly from T_{REG}N to T_{REG}NLM to T_{REG}CM to T_{REG}EM. The same pattern of CD31 expression was observed in conventional memory subset counterparts (Fig. 2E). Ki-67, a proliferation marker used to identify dividing cell populations, showed an inversely expressed profile with the highest expression by EM > CM > NLN/SCM > N T_{REG} cells (Fig. 2F). Recent TCR engagement was analyzed by CD134 (OX-40) expression, revealing highest expression among CM and EM, followed by NLM/SCM and very low expression by N (Fig. 2G) of both T_{REG} and T_{CONV} cells.

Next, we assessed the transcriptional expression levels of CD45RA and CD45RO for discriminating differentiation stages. CD45RO mRNA expression, a marker of memory T cells, is lowest in T_{REG}N and T_{REG}NLM cell subsets, increases in T_{REG}CM, and reaches its highest level in T_{REG}EM (Fig. 2H). CD45RA, expressed on naive CD4⁺ T cells, exhibits the highest mRNA expression in naive T_{REG} and is only slightly lower in T_{REG}NLM cells, and almost absent in T_{REG}CM and T_{REG}EM cells (Fig. 2I). As thymus-derived activated T_{REG} cells were reported to express the cytolytic marker granzyme A, we elected to examine its transcriptional expression and found that while normalized mRNA expression levels were generally low, the effector T_{REG} cells displayed the highest expression levels, followed my T_{REG}CM, T_{REG}NLM, and lowest expression by T_{REG}N cells (Fig. 2J). The ceramide synthase 6 (CERS6), which was demonstrated to play a role in inhibiting T cell activation and differentiation and promoting cellular quiescence by regulating intracellular ceramide production was expressed most abundantly at the transcriptional level in naïve T_{REG} cells followed by markedly lower levels in T_{REG}NLM, T_{REG}CM and lowest levels of *CERS6* in T_{REG}EM (Fig. 2K). While KLRG1 has traditionally been associated with a senescent state in effector T cells, we observe increased mRNA expression in early differentiated T_{REG}NLM and T_{REG}N subsets, which gradually decreases as differentiation progresses (Fig. 2L).

Next, we assessed the suppressive capacities of *ex vivo* isolated T_{REG}, as functionality rather than phenotypic markers defines the T_{REG} lineage, with none of these markers being exclusive to T_{REG}. To this end, we employed an *in vitro* suppression assay that measures the proliferation of autologous responder T cells (T_{RESP}) when co-cultured with varying ratios of T_{REG}.(19) All T_{REG} cell subsets exhibited suppressive capabilities in inhibiting the proliferation of T_{RESP} after 96 hours of co-culture. T_{REG}N LM and CM cells were particularly effective in suppressing T_{RESP} cell proliferation, specifically at ratios of 1:1 and 2:1, respectively. Naive T_{REG} demonstrated the lowest suppression capacities (Fig. 2M).

Taken together, the comprehensive phenotypic characterization of T_{REG} subsets demonstrates distinct phenotypic and functional variability, including marker expression, cytokine production, and suppressive capacities.

**The clonal TCR diversity and FoxP3-locus methylation analyses provide insights into the identity and lineage stability of the distinct T_{REG} memory-differentiation subsets.**

Subsequently, we conducted an analysis of the T cell receptor (TCR) repertoire to study the diversity (clonality) of T cell lineages during their differentiation process to gain further insight into the pathway of T_{REG} cell differentiation. Of all T_{REG} subsets, T_{REG}EM, being the furthest differentiated T_{REG} subset, demonstrated the greatest clonality (0.1027), representing the most oligoclonal TCR repertoire (Fig. 3A). Although the clonality of the two donors analyzed showed slight divergence, it was found that T_{REG}CM exhibited a higher level of diversity in their TCR repertoire (0.0041), making them the population with the closest approximation to maximum diversity. Of note, both T_{REG}NLM and T_{REG}N subsets had remarkably low clonality measures, suggesting that these two populations have the highest TCR diversity among the samples analyzed. However, the T_{REG}NLM cell subset retained a small but considerably greater degree of clonality (0.0027), suggesting a more oligoclonal TCR repertoire compared with the T_{REG}N cell population (0.001) (Fig. 3A). Analyzing the productive frequency of the top 10 clones, it was observed that the T_{REG}EM subset exhibited higher frequencies for these top clones compared to the T_{REG}CM > T_{REG}NLM > T_{REG}N cell subsets (Fig. 3B).

To analyze for T_{REG} cell subset-specific lineage stability, the degree of selective TSDR demethylation of ex *vivo* T_{REG} sub-populations was evaluated. Conventional CD4⁺CD25⁻ T cells (non-T_{REG}), demonstrated to have a fully methylated, *i.e.* non-demethylated TSDR, were also analyzed as an assay-specific reference control. Conventional CD4⁺CD25⁻ T cells display a fully methylated TSDR (0.19% demethylation) (Fig. 3C). We observed a mean TSDR demethylation of 90.16% within the bulk T_{REG} population. Within the T_{REG} subsets, the central memory population presents with the lowest degree of TSDR demethylation (83.89%), followed by the further differentiated T_{REG}EM subset (89.32%). Notably, T_{REG}N exhibits the highest level of TSDR demethylation, reaching 95.03%. Following closely, T_{REG}NLM demonstrates a demethylation rate of 90.75% for the TSDR region (Fig. 3C). A progressive loss of DNA methylation from conventional T_{N}> T_{CM} > T_{EM}, that was associated with a proliferative history has been reported. To test whether this phenomenon is also found among the different T_{REG} subsets, we performed methylation analysis yielding data on highly methylated domains (HMD) and partially methylated domains (PMD). While the differences among HMD were less pronounced, we found a strong increase in weighted average methylation from T_{REG}EM to T_{REG}CM to T_{REG}N LM to T_{REG}N (Fig. 3D), consistent with data for conventional T cell subsets.

In summary, the TCR repertoire and TSDR demethylation analyses revealed distinct clonality patterns and lineage stability among the T_{REG} subsets, with T_{REG}EM being the most clonal, T_{REG}CM closest to maximum diversity, T_{REG}NLM and T_{REG}N having the highest TCR diversity, and TSDR demethylation increasing progressively from T_{REG}EM to T_{REG}CM to T_{REG}NLM to T_{REG}N, similar to conventional T cells.

### Single-cell transcriptome and surface epitope analysis (CITE-seq) confirms coherence of memory formation between CD4⁺ T cells and T_{REG} cells

Using canonical surface markers to define T_{REG} cell differentiation stages and subsets, we found phenotypic, functional, clonal, and epigenetic coherence in the shaping of memory formation to conventional CD4⁺ T cells (Figs. 1-3). However, this definition is based on a sparse set of surface proteins, prompting us to validate this strategy with an unbiased approach using single-cell transcriptome analysis. Furthermore, we combined this analysis with cellular indexing of surface epitopes (CITE-seq) to validate the expression of surface markers defining T cell differentiation stages with transcriptomes for unequivocal confirmation. To this end, we isolated CD4⁺ T cells and donor-matched FACSorted T_{REG} cells from 7, respectively 5 healthy donors and subsequently analyzed the cells by CITE-seq. In order to uncover transcriptomic relationships among the individual cells and quantify for similarities, we performed Uniform Manifold Approximation and Projection for Dimension Reduction (UMAP) and unbiased clustering using the Louvain method for community detection (Fig. 4). Employing oligonucleotide-barcoded antibodies, we manually gated each CD4⁺ and T_{REG} subset according to the strategy presented in Fig. 1 and compared each subset to an unbiased transcriptome analysis. Black overlaying dots depict location within the UMAP, highlighting distinct transcriptomic features specific for each gated subset (Fig. 4A-H). Each CD4⁺ and T_{REG} cell subset can be recovered at distinct locations throughout the UMAP, thereby highlighting separate albeit slightly overlapping transcriptomic identities with proximity of the TN and T_{REG}N cells. The considerable overlap between CM and EM subsets, as defined by the presence or absence of CCR7 surface staining, hints towards a greater heterogeneity within the effector memory compartments. In addition, the T_{REG}NLM are intermediate between the T_{REG}N and memory profiles, signifying signatures of both compartments. The profiles of most differentiated T_{REG} cell subsets are positioned distal to the T_{REG}N / TN cell signature. Furthermore, the canonical bona fide T_{REG} cell markers can be assigned to each T_{REG} cell subset of Fig. 4 A-H (Fig. 4I: FOXP3, J: IL2RA, K: IL7R).

Unsupervised clustering (quantification) of bulk sorted CD4⁺ T cells together with T_{REG} cells is consistent with gated subsets (data not shown) and shows separation of the individual naive, effector and memory subsets, particularly in the T_{REG} cells. Eight distinct cell clusters were identified, comprising CD4⁺ T cells (clusters 5 and 8) and T_{REG} cells (clusters 1-4, 6-7) (data not shown). Top differentially expressed genes between gated subsets are summarized in the heatmap in Figure 4L and between unbiased identified clusters (data not shown). RNA transcripts of genes associated with effector function (e.g., CD40LG, KLRB1, CCL5, GZMA, GZMK) were upregulated in manually gated CD4⁺ T_{CM} and T_{EM} subsets (Fig. 4L). Likewise, T_{N}, T_{SCM}, T_{REG}N and T_{REG}NLM collectively showed strong expression of transcripts associated with migration to secondary lymphoid organs (SELL), survival, and homeostatic proliferation, which was less pronounced for subsets of memory type T cells. T_{REG}CM and T_{REG}EM subsets were specifically characterized by upregulation of HLA-D-types mRNA. In summary, we validated the surface markers defining T cell differentiation stages employing unbiased single-cell transcriptome analysis, providing unequivocal confirmation of their identities. The T_{REG} cell subsets exhibit distinct profiles, with a degree of overlap in the naive and memory-like T_{REG} cells, and the more differentiated T_{REG} cell subsets were positioned distal to the T_{REG}N/T_{N} cell signature, signifying the progression of differentiation.

### T_{REG} memory-differentiation subsets exhibit distinct temporal expansion potential in vitro

We set out to investigate the *in vitro* differentiation pattern of T_{REG} subsets following activation, with a focus on its relevance for adoptive T_{REG} therapy. To this end, we studied the expansion potential, changes in phenotype, production of proinflammatory cytokines under different conditions, suppression ability, and lineage stability. Since naive-like memory T_{REG} cells share the same protein marker combination as conventional T_{REG} cells, which are known to possess enhanced proliferation and self-renewal capacity compared to other conventional T-cell subsets, another objective was to determine whether T_{REG}NLM cells exhibit similar characteristics. To this end, the different T_{REG} subsets were sorted using FACS (Fig. 5A, B) and expanded using weekly aCD3/CD28 microbead bead stimulations simulating antigen-mediated activation (Fig. 5C). In the initial phase of expansion and up to week 3, the T_{REG}NLM subset showed greatest expansion, followed by T_{REG}N, T_{REG}CM and T_{REG}EM cells (Fig. 5D). Starting from week 3, T_{REG}CM exhibit the highest proliferative capacity, followed by T_{REG}NLM and bulk T_{REG}, with the latter two showing similar levels of expansion (Fig. 5E). Notably, the isolated T_{REG}EM cells exhibited the lowest level of expansion (Fig. 5E). Between weeks 4 and 7, all T_{REG} subsets, except for T_{REG}EM cells, either reached a plateau or experienced a decline in fold expansion.

**T_{REG} subsets exhibit distinct characteristics during expansion, with T_{REG}EM displaying loss of CD25/FoxP3, a central memory phenotype, increased cytokine production, reduced suppression, and TSDR methylation.**

We next examined the T_{REG} cell subsets phenotypically and functionally at different time points after *in vitro* expansion. Throughout a long-term expansion period of seven weeks, all T_{REG} cell subsets except T_{REG}EM cells stably retained their initial CD25⁺FoxP3⁺ expression (Fig. 6A). Approximately 40% of T_{REG}EM lost expression of CD25 and FoxP3 at week 7 post expansion (Fig. 6A). We next monitored potential changes in the initial naïve/memory phenotype after FACSorting at culture initiation for 7 weeks using their CD45RA and CCR7 expression. During the initial 3 weeks of expansion, T_{REG}NLM (90%) and T_{REG}N (80%) lost their initial phenotype of CD45RA⁺CCR7⁺ to the greatest extent to acquire a central memory-like phenotype (CD45RA⁻CCR7⁺). T_{REG}EM subsets underwent a phenotype transition to a central memory phenotype characterized by CD45RA⁻CCR7⁺ (approximately 40% of cells), whereas all other T_{REG} cell subsets, apart from T_{REG}EM cells, reverted back to their original phenotype between weeks 3 and 7 (Fig. 6I).

It is highly desirable for clinical-grade T_{REG} cell products to not produce pro-inflammatory cytokines, such as IFNy and IL-2, upon TCR-specific stimulation. This absence of cytokine production is considered a central release criterion for ensuring the stability of the cellular product. Following 3 weeks of expansion, the production of IFNγ and IL-2 within T_{REG} subsets was minimal (below 2.0% for IFNγ and 1.21% for IL-2 in T_{REG}CM), except for T_{REG}EM, which exhibited IFNγ and IL-2 production of 6.19% and 3.10%, respectively (Fig. 6C, D). From week 3 to week 7, the proportion of IFNγ producers increased in all memory subsets, but remained below 10% for each subset except T_{REG}EM (Fig. 6C). Taken together, the production of IFNy and IL-2 by all T_{REG} subsets was negligible until week 3 of expansion. However, thereafter, there was a notable rise in the proportion of IFNγ producers specifically among the T_{REG}EM subset, which correlated with the loss of their initial phenotype and decreased expression of FoxP3/CD25 (Fig. 6A-D).

Next, we assessed the suppressive capacities of the *in vitro* expanded T_{REG} subsets. After 3 weeks of *in vitro* expansion, the T_{REG} subsets, including T_{REG}Bulk, T_{REG}CM, T_{REG}NLM, and T_{REG}N co-cultured with autologous T_{RESP} at a 1:1 ratio, exhibited a great ability to suppress 80-90% of both CD4⁺ and CD8⁺ T_{RESP}. However, T_{REG}EM showed a lower level of suppression, with only 57.99% suppression of CD4⁺ T_{RESP} and 48.86% suppression of CD8⁺ T_{RESP} (Fig. 6E). Furthermore, when the T_{RESP}:T_{REG} ratio was increased to 10:1, T_{REG}N LM demonstrated the highest level of suppression in terms of T_{RESP} proliferation among all T_{REG} subsets, followed by T_{REG}N and T_{REG}CM and T_{REG}Bulk (Fig. 6F). We next examined the epigenetic stability of the TSDR following the initial 3 weeks of expansion (Fig. 6G). T_{REG}Bulk, T_{REG}NLM and T_{REG}N demonstrated the greatest TSDR demethylation, whereas in comparison, T_{REG}CM presented 20% lower demethylation. Only 13% of the T_{REG}EM TSDR was demethylated at this timepoint. During weeks 5 and 7, the differences in TSDR demethylation among the T_{REG} subsets remained consistent with the findings from week 3. The less differentiated T_{REG} subsets exhibited the highest degree of lineage stability, while T_{REG}EM demonstrated complete methylation of the TSDR (Fig. 6G).

Finally, we investigated the effect of depleting the EM-like T_{REG} subset prior to manufacturing the cell product in order to test the impact of removing this small but instable subset on the functionality of the resulting T_{REG} product. For this purpose, we employed a FACSort to deplete EM cells (CCR7⁻CD45RA⁻) from the initial T_{REG} population, and then compared these cultures with donor-matched bulk T_{REG} cells (Fig. 7A). Over a 21-day period, we observed a modest increase in the expansion of T_{REG}EM-depleted cell product compared to bulk T_{REG} subsets that retained the EM population. The percentage of FoxP3/CD25 positive cells in the final product on day 21 showed no significant difference on average. However, the bulk T_{REG} subset products were more heterogeneous to one another with respect to their final phenotype, frequencies at FoxP3/CD25, and TSDR demethylation (Fig. 7C-E). Depletion of the T_{REG}EM population reduces the overall heterogeneity between T_{REG} cell products from different donors. Thus, depletion of the T_{REG}EM subset permits the generation of stable T_{REG} cell products in different donors without major impact on yield. Lastly, we investigated the propensity of T_{REG} cells to dedifferentiate into T_{H17} cells in a strong inflammatory environment using a T_{H17} differentiation protocol based on Koenen et al. (18) To test the impact of IL-6 as a proposed key driver of dedifferentiation, we created a targeted gene knock-out to disrupt the IL-6 receptor complex (IL-6R + gp130) in bulk and CCR7⁺ enriched T_{REG} cells (Fig. 8A-C). Whereas > 5% of wild-type bulk T_{REG} produced IL-17 post IL-6 challenge, IL-6Ra or gp130 knock-out bulk T_{REG} showed a markedly reduced expression of about 1% and 2%, respectively. Remarkably, we found that our sorting strategy for CCR7⁺ T_{REG} (depleting T_{REG}EM) prior to *in vitro* T_{H17} induction culture effectively prevents T_{H17} production in unstimulated and IL-6 stimulated cultures (Fig. 8D).

Taken together, during *in vitro* expansion, the T_{REG} subsets showed differential phenotypic changes, cytokine production, suppressive capacities, and epigenetic stability, with T_{REG}EM displaying a loss of CD25 and FoxP3 expression, acquisition of a central memory phenotype, increased production of IFNγ and IL-2, decreased suppressive ability, high lineage instability in an inflammatory environment, and complete methylation of the TSDR, highlighting the importance of considering these factors for the development of clinical-grade T_{REG} products.

### Discussion

Therapeutic applications of T_{REG} in inflammatory diseases, transplantation and gene therapies hold promise, and recent advancements in CAR development and genome editing have been employed to improve their specificity and functionality. However, several challenges remain, including donor variability, manufacturing failures and the need for prolonged expansion periods. Furthermore, crucial safety considerations continue to pertain, such as retrieving the optimal T_{REG} cell subset and the necessity to eliminate impurities while preserving T_{REG} integrity. The central purpose of our study was to investigate the characteristics of thymic-derived T_{REG} subsets, with respect to differentiation stages, to the bulk *in vitro* stimulated T_{REG} cell products that are eventually infused into patients in clinical trials. We examined T_{REG} subset derivation, fate, epigenomic stability, transcriptome, TCR repertoires, and function for clinical-grade manufacturing. Each subset displayed unique functional features, epigenomic lineage stability within the FoxP3 locus, surface marker expression, transcriptome and TCR diversity. We validated the surface markers that characterize canonical stages of T cell differentiation using an unbiased single-cell transcriptome analysis and corroborated the surface markers defining T-cell differentiation stages, with transcriptomes providing unequivocal confirmation of their identities. Earlier differentiated memory T_{REG} populations showed superior suppression of autologous responder T cell proliferation compared to effector memory-like T_{REG}. These memory T_{REG} populations also exhibited the highest proliferative capacity regardless of TCR stimulation duration and frequency. They maintained a stable phenotype post-activation and had high demethylation of the TSDR over time. In contrast, effector memory-like T_{REG} showed decreased TSDR demethylation and immunosuppressive function while producing effector cytokines. Despite this, these effector-like T_{REG} products retained FoxP3 expression and phenotypically resembled thymic-derived T_{REG}. By depleting the effector memory-like T_{REG} subset prior to manufacturing, we successfully produced stable T_{REG} products with consistent characteristics, exemplified by reduced conversion to T_{H17} cells, showcasing increased stability against inflammatory triggers.

Successful T_{REG} cell therapy depends on multiple cell- and patient-dependent factors. Among these, the T cell subset origin of the final T cell product infused may be critical to the outcome by contributing to post-infusion cell expansion, suppressive function, lineage stability, and long-term persistence. However, although the phenotype such as FoxP3/CD25 positivity of the final T_{REG} cell product infused is often described, the T_{REG} cell subsets in relation to its functional and epigenetic properties is rarely investigated, so that functional dissection of the infusion product remains a prerequisite to better understand and correlate therapy outcome with T cell product input. As functional testing remains challenging(19), surrogate release criteria such epigenetic integrity (TSDR demethylation) or the absence of cytokine production is considered central for ensuring the stability and authenticity of the T_{REG} product. Accordingly, it must be precluded that potential contaminating effector T cells or instable T_{REG} subsets are comprised in the cell product.

Immunological memory is essential for effector T cells to respond efficiently to pathogens upon secondary infection. While the benefits of memory in pro-inflammatory cells are evident, memory T_{REG} cells' significance is less clear. They are believed to reduce tissue damage during heightened pro-inflammatory responses and support fetal tolerance during pregnancy. Recent studies confirm the persistence of antigen-specific T_{REG} cells with potent immunosuppressive properties even after antigen elimination. However, definitive markers are lacking, hindering the study of memory T_{REG} cells. To understand their function in health and disease, comprehensive transcriptional profiling and detailed examination of epigenetic and metabolic signatures are needed. Using a diverse set of methods including unsupervised clustering of single-cell transcriptome and surface epitope analysis in conjunction with canonical surface marker staining in flow cytometry, we identified T cell differentiation stages within the T_{REG} cell lineage enabling consistent separation of naive, effector, and memory T_{REG} cell subsets. Our findings suggest a strong correlation and coherence for memory formation between T_{REG} subsets and their CD4⁺ T_{CONV} counterparts, implying potential similarities in their differentiation pathways. Our results support the presence of a Tscnn-like population within the CD4⁺ T cell population, similar to what was originally described for CD8⁺ T cells (20, 21). In addition, we discovered a previously unidentified population among the CD4⁺ T_{REG} subsets, which we denominated naive-like memory T_{REG}, and whose advantageous properties in terms of stability and suppressive capacity we deem attractive for immunotherapeutic applications. These T_{REG}NLM had slightly higher clonality than T_{REG}N, suggesting their allegiance and identity to the memory T cell pool, whereas the further differentiated T_{REG} subsets (T_{REG}CM and T_{REG}EM) had the least diverse repertoire potentially due to clonal expansion after antigen encounter, similar to conventional memory T cells. Intriguingly, we found a strong increase in weighted average methylation from late-differentiated T_{REG} to naive T_{REG}, as reported for T_{CONV}, supporting a linear differentiation from T_{REG}N to T_{REG}NLM to T_{REG}CM to T_{REG}EM. Our findings demonstrate that T_{REG}N that also gradually declined with age, concurrent with accumulating T_{REG}EM frequencies with age. In general, our results indicated that similar to conventional memory CD4⁺ T cells, memory-like T_{REG} subsets (T_{REG}CM and T_{REG}EM) showed an increase with advancing age. We further observed increasing expression of FoxP3 and CD25 with increased differentiation of T_{REG} memory subsets, indicating recent activation leading to T_{REG} differentiation. However, despite the initial high FoxP3 expression in all subsets, TSDR demethylation decreased in T_{REG}EM as early as week 3 along FoxP3 protein expression, indicating a loss of T_{REG} stability and suppressive properties during expansion. This could potentially be related to the emergence of so-called exT_{REG}, which are pathogenic in autoimmune settings and produce IFN-γ. We therefore suggest depleting T_{REG}EM prior to T_{REG} manufacture to increase T_{REG} product stability and safety. In fact, our findings demonstrate that depleting the EM-like T_{REG} subset prior to manufacturing the cell product leads to a more homogeneous and stable T_{REG} cell product. The expansion of T_{REG}EM-depleted cells was modestly increased, suggesting that the presence of EM-like T_{REG} might hinder the overall expansion potential of T_{REG} cells during *in vitro* culture. However, the percentage of FoxP3/CD25 positive cells in the final product was not significantly affected, indicating that T_{REG}EM depletion does not compromise the maintenance of the regulatory phenotype. The enhanced stability of T_{REG}EM-depleted cell products appears particularly important, as it ensures consistent and reliable outcomes across different donors. Heterogeneity in T_{REG} products from various donors can lead to variable therapeutic responses, which may limit the efficacy of T_{REG}-based therapies. Previous studies have shown that T_{REG} can lose their suppressive function and acquire pro-inflammatory characteristics, such as T_{H17} -like features, under certain conditions. Remarkably, we found that a sorting strategy for CCR7⁺ T_{REG}, which effectively depleted T_{REG}EM cells, prior to *in vitro* T_{H17} induction culture, successfully prevented dedifferentiation of T_{REG} cells. This result indicates that the presence of EM-like T_{REG} cells may contribute to the dedifferentiation of T_{REG} under inflammatory conditions, and their removal can preserve the regulatory function of T_{REG} cells even in a pro-inflammatory environment.

First clinical trials are launched, which exclusively expand T_{REG}N-comprising (and indeed T_{REG}NLM) CD45RA⁺ T_{REG} for genetic engineering purposes based on their reported advantage in stability, however, missing a relatively stable T_{REG} subset with the highest expansion capacity, *i.e.* T_{REG}CM. In fact, this subset showed lower TSDR demethylation than T_{REG}N and T_{REG}NLM post expansion. Nevertheless, a manufacturing process achieving sufficient numbers for clinical applications starting with solely naive T_{REG} may not be feasible in every patient, particularly in the elderly. The expansion rate of T_{REG} subset appeared to be low although not negligible. Furthermore, it should be cautioned not to extend the expansion rate beyond 5 weeks, as this could lead to functional and phenotypic instability. *In vivo* expansion of bulk T_{REG} products can only be inferred, and *in vivo* tracking studies are needed to provide insight.

Overall, our study address challenges in identifying optimal T_{REG} subsets for stable and potent suppressive cells, as impurities and phenotypic instability hinder edited T_{REG} product development, necessitating precautions during manipulations. We examined T_{REG} characteristics ex *vivo* and post expansion, revealing that earlier differentiated memory T_{REG} populations outperformed late-differentiated effector memory-like T_{REG} in suppressive capacity, higher proliferative capacity, stable phenotypes, and strong demethylation of the TSDR over time, highlighting the importance of considering T_{REG} subset composition before expansion in adoptive T_{REG} immunotherapy to maintain lineage stability in the final cell product. Next, development of a GMP-protocol using the proposed cell separation and clinical studies are needed to further detangle the relevance of our finding for adoptive T_{REG} therapy.

### Materials and Methods

### Blood sampling and PBMC isolation

Venous blood samples were collected from 53 healthy donors (30 female/23 male, 24-82 years), who had given their written informed consent. The study was approved by the Charité - Universi-tätsmedizin Berlin Ethics Committee. PBMCs were isolated using Biocoll (Biochrom) gradient centrifugation.

### FACSorting and expansion of T_{REG} subsets

Freshly isolated PBMCs were enriched for CD4+ T cells via positive selection by magnetically activated cell separation (CD4 MicroBeads, Miltenyi Biotec) and stained with monoclonal antibodies (CD4, SK3; CD25, 2A3, BD Biosciences; CD127, R34.34, Beckman Coulter; CD45RA, HI100; CCR7, G043H7; CD45RO, UCHL1; CD62L, DREG-56; CD95, DX2; all Biolegend unless stated) for subsequent T_{REG} subset isolation by FACSorting. For specific experiments, the T_{REG}EM compartment has been depleted from bulk T_{REG} by FACSorting to yield CD4+CD25+CD127- T_{REG} and CD4+CD25+CD127-CCR7+ T_{REG}-EM subsets. FACSorted T_{REG} subsets were suspended in X-Vivo15 (Lonza) medium supplemented with 10% FCS (PAA), 100 U/mL penicillin, 100 µg/mL streptomycin (both Biochrom), 500U/mL rhlL-2 (Proleukin S, Novartis) and 100µM Rapamycin (Rapamune, Pfizer Pharma GmbH) and placed into humidified incubators at 37°C and 5% CO2. On day 1, T_{REG} expansion beads (Miltenyi Biotec), particles loaded with antiCD3/CD28 antibodies, were added at a bead-to-cell ratio of 4:1. On day 7 and 14, the cells were re-stimulated at a bead-to-cell ratio of 1:1 and the medium containing all above-mentioned supplements. Additionally, cells were split, and medium changed when indicated by cell density and/or medium color change.

### Phenotype and cytokine profile analysis

Ex vivo isolated PBMC and expanded T_{REG} subsets were analyzed for their phenotype and pro-inflammatory cytokine profile. For detecting intracellular cytokine production, cells were cultured with 10ng/mL PMA (phorbol myristrate acetate) and 1µg/mL lonomycin for 4 hours, after which 4µg/mL Brefeldin A (all Sigma-Aldrich) was added for further 2 hours.

To define memory subsets, T cells were extracellularly stained für surface markers CD25 (2A3, BD Biosciences), CD45RA (HI100), CCR7 (G043H7), CD45RO (UCHL1), CD62L (DREG-56), CD31 (WM59) and CD134 (Ber-ACT35), permeabilized/fixed with Foxp3/Transcription Factor Staining Buffer Set (eBioscience) and stained intracellularly for CD3 (OKT3), CD4 (SK3), CD8 (RPA-T8), CD95 (DX2), FoxP3 (259D/C7, BD Biosciences), Ki-67 (Ki-67), IFNγ (4S.B3) and IL-2 (MQ1-17H12) (all Biolegend unless stated). Data were acquired and analyzed on a LSR II Fortessa flow cytometer and by using FlowJo Version 10 software (Tree Star).

### Responder T cell proliferation suppression assay

For ex vivo FACSorted T_{REG} subsets, autologous CD4+CD25- effector T cells were used as both, responder cells (T_{RESP}) and negative control to ensure T_{REG} integrity and functionality. For expanded T_{REG} subsets, freshly isolated and enriched autologous CD3 T cells (Human T cell Enrichment Cocktail, Stemcell) were used. Responder cells were stained with 10µM CFSE (Sigma-Aldrich) and co-cultured at different T_{RESP}:T_{REG} ratios. Cells were stimulated with anti-CD3/28-coated microbeads (T_{REG} Suppression Inspector, Miltenyi Biotec) at a cell/bead ratio of 1:1 and incubated at 37°C for 96 hours. Subsequently, cells were extracellularly labeled with CD3 (OKT3), CD4 (SK3), CD8 (RPA-T8) (all Biolegend) and LIVE/DEAD Fixable Blue Dead Cell Stain (Invitro-gen). Data were acquired and analyzed on a LSR II Fortessa flow cytometer and by using FlowJo Version 10 software (Tree Star). Proliferation was assessed by CFSE dilution and percentage suppression of proliferation was calculated by relating the percentage of proliferating T_{RESP} in the presence and absence of T_{REG}, respectively.

### TSDR methylation analysis

Genomic DNA from ex vivo FACSorted or expanded T cell subsets was extracted (QlAamp DNA blood mini kit, Qiagen). Each real-time PCR reaction contained a minimum of 60 ng bisulfite-treated (EpiTect, Qiagen) genomic DNA or a respective amount of plasmid standard, 10 uL FastStart universal probe master (Roche Diagnostics), 50 ng/mL lamda DNA (New England-Biolabs), 5 pmol/mL methylation or nonmethylation-specific probe and 30 pmol/mL methylation or nonmethylation-specific primers. The samples were analyzed in triplicates on an ABI 7500 cycler. For some experiments genomic DNA was isolated using AllPrep DNA/RNA Mini Kit (Qiagen) or Zymo's Quick-DNA MicroPREP Kit (Zymo Research), following manufacturer's instructions. 200-250 ng of genomic DNA was used as input for bisulfite conversion, which was performed with Zymo's EZ DNA Methylation-Gold Kit (Zymo Research) or Zymo's EZ DNA Methylation Kit (Zymo Research). In specific experiments, methylation analysis was performed with the Infinium Methylation EPIC Kit (Illumina EPIC-8 BeadChip) following the manufacturer's instructions. Bead Chips were imaged on Illumina's Microarray Scanner iScan. Amplicons were purified with QIAquick PCR Purification Kit (Qiagen 28106) and normalized to 20ng/µL, for sequencing by Genewiz/ Azenta.

### Quantitative real-time PCR

Total RNA of FACSorted T_{REG} subsets was extracted using NucleoSpin RNA II Kit (Macherey-Nagel) following manufacturer's instructions. Up to 1 µg RNA was used for cDNA synthesis according to the QuantiTect Reverse Transcription Kit (Qiagen) manual. hHPRT was used for the normalization of expression levels. Quantitative real-time PCR analysis was performed using TaqMan Universal PCR Master Mix and ready-to-use detections system, containing FAM-TAMRA-labeled probes that were purchased from Applied Biosystems (ABI). All expression levels were analyzed in duplicate using ABI Prism 7500 Sequence detections system and associated software (all from ABI). All expression levels were calculated relative to hHPRT expression levels.

### TCR next-generation sequencing

CDR3 sequencing was performed on the ImmunoSEQ platform at Adaptive Biotechnologies as previously described by Robins et al.(22) and Sherwood et al. (23). Sequences that did not match CDR3 sequences were removed from the analysis. For further analysis, the standard algorithm as developed by Adaptive Biotechnologies and previously described by Yousfi Monod et al. (24) was used. Genomic DNA was isolated using QlAamp DNA Mini Kit from Qiagen according to manufacturer's instructions.

### CITEseq analysis

Ex vivo FACSorted T_{REG} and conventional CD4+ T cells were labelled with anti-human TotalSeq-C hashtag antibodies (Biolegend) allowing the pooling of samples followed by labelling with anti-human TotalSeq-C antibodies (Biolegend) targeting a panel of extracellular proteins. Antibody and transcriptome libraries were prepared by using the Chromium Single Cell 5' Library & Gel Bead Kit as well as the Single Cell 5' Feature Barcode Library Kit version 1.1 (10x Genomics) following manufacturer's instructions. Qubit HS DNA assay kit (Life Technologies) was used for library quantification, and fragment sizes were obtained using the 2100 Bioanalyzer using the High Sensitivity DNA Kit (Agilent). Sequencing was performed on a NextSeq500 device (Illumina) using High Output v2 Kits (150 cycles) with the recommended sequencing conditions for 5' GEX and feature barcode libraries (read1: 26 nt, read2: 98 nt, index1: 8 nt, index2: n.a.,1 % PhiX spike-in). Raw sequence reads were processed using cellranger-5.0.0, including the default detection of intact cells. Mkfastq and count were used in default parameter settings for demultiplexing and quantifying the gene expression. Refdata-cellranger-hg19-1.2.0 was used as reference. Raw UMI-counts were further processed and analyzed using R 4.3.1 according to the osca workflow by Lun et al.91 including normalization, filtering of low-quality cells, clustering and UMAP dimensionality reduction. Differentially expressed genes between gated subsets and clusters, respectively, were identified using the findMarkers and multiMarkerStats functions.

### Reduced Representation Bisulfite Sequencing (RRBS)

Libraries for RRBS were prepared according to the protocol previously described with minor modifications (25). Briefly, fresh frozen cell pellets -30,000 - 130, 000 cells) were lysed by overnight incubation at 55° C after adding 16 µl lysis buffer (10mM TrisHCl, 5mM EDTA) and 4 µl proteinase K (1 mg/ml; Merck Millipore, Burlington, USA). Proteinase K was then inactivated by incubation with 2.8 µl of 8.14mM Pefabloc SC (Merck Millipore) for 1 h at room temperature. Then samples were cut overnight at 37° C by adding 1µl of 50 U/µl Haelll (NEB, Ipswich, USA), 3 µl of 10x CutSmartbuffer (NEB) and 3.2 µl of nuclease-free water.

Blunt-end DNA fragments were subjected to A-tailing with Klenow fragment exo- (50 U/µl; NEB) for 30 min at 37° C, followed by inactivation for 20 min at 75° C. Methylated sample-specific sequencing adaptors were ligated using T4-ligase (NEB) followed by bisulfite conversion with the EZ-DNA methylation Gold kit (Zymo research, Irvine, USA). Converted samples were PCR amplified for 15-18 cycles and then purified with AMPure XP beads (Beckham Coulter, Brea, USA). NGS libraries were then sequenced for-ca. 30 - 70 mio of 100 bp single reads on an HiSeq 2500 platform (Illumina, San Diego, USA). Raw reads were trimmed with the Trim Galore! wrapper (http://www.bioinformatics.babraham.ac.uk/projects/trim_galore) in RRBS mode. Reads were mapped to hg38 genome using the BWA wrapper methylCtools (v0.9.2) (Hovestadt et al., 2014) and DNA methylation calling was performed as described in Durek et al. 2018 (17). Weighted average methylation was calculated and plotted across PMDs and HMDs obtained from TEM sample from Durek et al. 2018 (17).

### Gene editing of IL-6 signaling pathway

Knock-out of IL6R and gp130 was performed by electroporation of CRISPR-Cas9 ribonucleoprotein complexes as previously described (26). Briefly, regulatory T cells were expanded until day 7-8, harvested and residual beads were depleted by strong magnet stand. Single guide RNAs and recombinant Cas9 protein (IDT) were pre-complexed for 10-15 minutes at room temperature. The electroporation was performed with the P3 primary cell Kit (Lonza) and nucleofection program "EH-115" on a Lonza 4D-nucleofector. After electroporation, the cells were rescued in pre-warmed T_{REG} medium and carefully transferred back into culture dish after a 10-minute resting step at 37°C and 5% CO2. T_{REG} were restimulated with expansion beads after an overnight rest.

### Analysis of STAT3 phosphorylation after IL-6 exposure

T_{REG} were rested for 24 hours in cytokine-free T_{REG} medium. 1x106 T_{REG} cells were first stained with Live/Dead UV Fixable Dye (ThermoFisher) according to the manufacturer's recommendation. Then, the T_{REG} were resuspended either in pre-warmed medium containing IL-6 (100 ng/ml) and soluble IL-6 receptor (10 ng/mL) or no cytokine for 30 minutes. Immediately at the end of incubation, the T_{REG} were fixed with pre-warmed Fixation Buffer at 37°C (Biolegend) and incubated for 15 minutes at 37°C. Then, T_{REG} were washed and permeabilization with ice-cold True-Phos Permeabilization Buffer (Biolegend) at -20°C for 60 minutes. During permeabilization and fixation, T_{REG} were stained intracellularly with monoclonal antibodies for CD3 (1:25, anti-CD3 PacBlue, Beckman Coulter), CD4 (1:25, anti-CD4 PE, Beckman Coulter Phosphor phospho-STAT3 (1:100, anti-pSTAT3 Alexa Fluor 647, Biolegend) in 1xPBS.

### T_{H17} -differentiation assay

To evaluate the propensity of T_{REG} to de-differentiate into T_{H17} cells, an T_{H17} differentiation assay was performed using a protocol adapted from a previous study (18). Briefly, expanded T_{REG} cells (see above) were exposed to irradiated allogeneic PBMCs in the presence of inflammatory cytokines IL-1beta (20ng/mL), IL-2 (500 U/mL), IL-6 (100 ng/mL) over seven days. As controls, T_{REG} were expanded with anti-CD3/28 expansion beads (Miltenyi) and rhlL-2 (500U/mL, Proleukin S, Novartis) containing T_{REG} medium. Then, different cultivated T_{REG} were re-stimulated with PMA/lonomycin (as described above) in IL-6 containing medium (100 ng/mL). Intracellular cytokine production was assessed as described above by including an anti-IL-17A antibody (1:100, anti-IL-17A PE, BioLegend).

### References

1. Sakaguchi, S., T. Yamaguchi, T. Nomura, and M. Ono. 2008. Regulatory T Cells and Immune Tolerance. Cell 133: 775-787.
2. Roemhild, A., N. M. Otto, G. Moll, M. Abou-EI-Enein, D. Kaiser, G. Bold, T. Schachtner, M. Choi, R. Oellinger, S. Landwehr-Kenzel, K. Juerchott, B. Sawitzki, C. Giesler, A. Sefrin, C. Beier, D. L. Wagner, S. Schlickeiser, M. Streitz, M. Schmueck-Henneresse, L. Amini, U. Stervbo, N. Babel, H.-D. Volk, and P. Reinke. 2020. Regulatory T cells for minimising immune suppression in kidney transplantation: phase I/Ila clinical trial. BMJ 371: m3734.
3. Landwehr-Kenzel, S., L. Müller-Jensen, J.-S. Kuehl, M. Abou-el-Enein, H. Hoffmann, S. Muench, D. Kaiser, A. Roemhild, H. von Bernuth, M. Voeller, M. Schmueck-Henneresse, B. Gruhn, U. Stervbo, N. Babel, H.-D. Volk, and P. Reinke. 2022. Adoptive transfer of ex vivo expanded regulatory T cells improves immune cell engraftment and therapy-refractory chronic GvHD. Mol. Ther. 30: 2298-2314.
4. Taams, L. S., M. Vukmanovic-Stejic, J. Smith, P. J. Dunne, J. M. Fletcher, F. J. Plunkett, S. B. Ebeling, G. Lombardi, M. H. Rustin, J. W. J. Bijlsma, F. P. J. G. Lafeber, M. Salmon, and A. N. Akbar. 2002. Antigen-specific T cell suppression by human CD4+CD25+ regulatory T cells. Eur. J. Immunol. 32: 1621-1630.
5. Fan, M. Y., J. S. Low, N. Tanimine, K. K. Finn, B. Priyadharshini, S. K. Germana, S. M. Kaech, and L. A. Turka. 2018. Differential Roles of IL-2 Signaling in Developing versus Mature Tregs. Cell Rep. 25: 1204-1213.e4.
6. Baecher-Allan, C., J. A. Brown, G. J. Freeman, and D. A. Hafler. 2001. CD4+CD25high Regulatory Cells in Human Peripheral Blood. J. Immunol. 167: 1245-1253.
7. Dieckmann, D., H. Plottner, S. Berchtold, T. Berger, and G. Schuler. 2001. Ex Vivo Isolation and Characterization of Cd4+Cd25+ T Cells with Regulatory Properties from Human Blood. J. Exp. Med. 193: 1303-1310.
8. Ng, W. F., P. J. Duggan, F. Ponchel, G. Matarese, G. Lombardi, A. D. Edwards, J. D. Isaacs, and R. I. Lechler. 2001. Human CD4+CD25+ cells: a naturally occurring population of regulatory T cells. Blood 98: 2736-2744.
9. Miyara, M., Y. Yoshioka, A. Kitoh, T. Shima, K. Wing, A. Niwa, C. Parizot, C. Taflin, T. Heike, D. Valeyre, A. Mathian, T. Nakahata, T. Yamaguchi, T. Nomura, M. Ono, Z. Amoura, G. Gorochov, and S. Sakaguchi. 2009. Functional Delineation and Differentiation Dynamics of Human CD4+ T Cells Expressing the FoxP3 Transcription Factor. Immunity 30: 899-911.
10. Polansky, J. K., K. Kretschmer, J. Freyer, S. Floess, A. Garbe, U. Baron, S. Olek, A. Hamann, H. von Boehmer, and J. Huehn. 2008. DNA methylation controls Foxp3 gene expression. Eur. J. Immunol. 38: 1654-1663.
11. Walter, E. A., P. D. Greenberg, M. J. Gilbert, R. J. Finch, K. S. Watanabe, E. D. Thomas, and S. R. Riddell. 1995. Reconstitution of Cellular Immunity against Cytomegalovirus in Recipients of Allogeneic Bone Marrow by Transfer of T-Cell Clones from the Donor. N. Engl. J. Med. 333: 1038-1044.
12. Zhou, X., S. L. Bailey-Bucktrout, L. T. Jeker, C. Penaranda, M. Martinez-Llordella, M. Ashby, M. Nakayama, W. Rosenthal, and J. A. Bluestone. 2009. Instability of the transcription factor Foxp3 leads to the generation of pathogenic memory T cells in vivo. Nat. Immunol. 10: 1000-1007.
13. Amini, L., J. Greig, M. Schmueck-Henneresse, H.-D. Volk, S. Bézie, P. Reinke, C. Guillon-neau, D. L. Wagner, and I. Anegon. 2021. Super-Treg: Toward a New Era of Adoptive Treg Therapy Enabled by Genetic Modifications. Front. Immunol. 11: 611638.
14. Kressler, C., G. Gasparoni, K. Nordström, D. Hamo, A. Salhab, C. Dimitropoulos, S. Tierling, P. Reinke, H.-D. Volk, J. Walter, A. Hamann, and J. K. Polansky. 2021. Targeted De-Methylation of the FOXP3-TSDR Is Sufficient to Induce Physiological FOXP3 Expression but Not a Functional Treg Phenotype. Front. Immunol. 11: 609891.
15. Lei, H., L. Kuchenbecker, M. Streitz, B. Sawitzki, K. Vogt, S. Landwehr-Kenzel, J. Millward, K. Juelke, N. Babel, A. Neumann, P. Reinke, and H. -D. Volk. 2015. Human CD45RA- FoxP3hi Memory-Type Regulatory T Cells Show Distinct TCR Repertoires With Conventional T Cells and Play an Important Role in Controlling Early Immune Activation. Am. J. Transplant. 15: 2625-2635.
16. WO 2022/034233 A1 IMMUNOSUPPRESSANT-RESISTANT T-CELLS FOR ADOPTIVE IMMUNOTHERAPY.
17. Durek, P., K. Nordström, G. Gasparoni, A. Salhab, C. Kressler, M. de Almeida, K. Bassler, T. Ulas, F. Schmidt, J. Xiong, P. Glažar, F. Klironomos, A. Sinha, S. Kinkley, X. Yang, L. Arrigoni, A. D. Amirabad, F. B. Ardakani, L. Feuerbach, O. Gorka, P. Ebert, F. Müller, N. Li, S. Frischbutter, S. Schlickeiser, C. Cendon, S. Fröhler, B. Felder, N. Gasparoni, C. D. Imbusch, B. Hutter, G. Zipprich, Y. Tauchmann, S. Reinke, G. Wassilew, U. Hoffmann, A. S. Richter, L. Sieverling, D. Consortium, H.-D. Chang, U. Syrbe, U. Kalus, J. Eils, B. Brors, T. Manke, J. Ruland, T. Lengauer, N. Rajewsky, W. Chen, J. Dong, B. Sawitzki, H.-R. Chung, P. Rosenstiel, M. H. Schulz, J. L. Schultze, A. Radbruch, J. Walter, A. Hamann, and J. K. Polansky. 2016. Epigenomic Profiling of Human CD4+ T Cells Supports a Linear Differentiation Model and Highlights Molecular Regulators of Memory Development. *Immunity* 45: 1148-1161.
18. Koenen, H. J. P. M., R. L. Smeets, P. M. Vink, E. van Rijssen, A. M. H. Boots, and I. Joosten. 2008. Human CD25highFoxp3pos regulatory T cells differentiate into IL-17-producing cells. Blood 112: 2340-2352.
19. Wendering, D. J., L. Amini, S. Schlickeiser, P. Reinke, H.-D. Volk, and M. Schmueck-Henneresse. 2019. The Value of a Rapid Test of Human Regulatory T Cell Function Needs to be Revised. Front. Immunol. 10: 150.
20. Gattinoni, L., E. Lugli, Y. Ji, Z. Pos, C. M. Paulos, M. F. Quigley, J. R. Almeida, E. Gostick, Z. Yu, C. Carpenito, E. Wang, D. C. Douek, D. A. Price, C. H. June, F. M. Marincola, M. Roederer, and N. P. Restifo. 2011. A human memory T cell subset with stem cell-like properties. Nat. Med. 17: 1290-1297.
21. Schmueck-Henneresse, M., R. Sharaf, K. Vogt, B. J. D. Weist, S. Landwehr-Kenzel, H. Fuehrer, A. Jurisch, N. Babel, C. M. Rooney, P. Reinke, and H.-D. Volk. 2014. Peripheral blood-derived virus-specific memory stem T cells mature to functional effector memory subsets with self-renewal potency. J. Immunol. (Baltim., Md: 1950) 194: 5559-67.
22. Robins, H. S., P. V. Campregher, S. K. Srivastava, A. Wacher, C. J. Turtle, O. Kahsai, S. R. Riddell, E. H. Warren, and C. S. Carlson. 2009. Comprehensive assessment of T-cell receptor beta-chain diversity in alphabeta T cells. Blood 114: 4099-107.
23. Sherwood, A. M., C. Desmarais, R. J. Livingston, J. Andriesen, M. Haussler, C. S. Carlson, and H. Robins. 2011. Deep Sequencing of the Human TCRγ and TCRβ Repertoires Suggests that TCRβ Rearranges After αβ and γδ T Cell Commitment. Sci. Transl. Med. 3: 90ra61.
24. Monod, M. Y., V. Giudicelli, D. Chaume, and M.-P. Lefranc. 2004. IMGT/JunctionAnalysis: the first tool for the analysis of the immunoglobulin and T cell receptor complex V-J and V-D-J JUNCTIONs. Bioinformatics 20: i379-i385.
25. Luetke-Eversloh, M., Q. Hammer, P. Durek, K. Nordström, G. Gasparoni, M. Pink, A. Hamann, J. Walter, H.-D. Chang, J. Dong, and C. Romagnani. 2014. Human Cytomegalovirus Drives Epigenetic Imprinting of the IFNG Locus in NKG2Chi Natural Killer Cells. PLoS Pathog. 10: e1004441.
26. Amini, L., D. L. Wagner, U. Rössler, G. Zarrinrad, L. F. Wagner, T. Vollmer, D. J. Wendering, U. Kornak, H.-D. Volk, P. Reinke, and M. Schmueck-Henneresse. 2021. CRISPR-Cas9-Edited Tacrolimus-Resistant Antiviral T Cells for Advanced Adoptive Immunotherapy in Transplant Recipients. Mol. Ther. 29: 32-46.

## Claims

1. A composition comprising CD4+CD25+CD127low regulatory T cells, wherein the regulatory T cells comprise:
• CD45RA+CD95- and CCR7+and/or CD62L+ naive regulatory T cells,
• CD45RO+and CCR7+and/or CD62L+ central memory regulatory T cells,
wherein the composition does not comprise CD45RO+CCR7-CD62L- effector memory regulatory T cells.

2. The composition of claim 1, wherein the composition further comprises CD45RA+CD95+ and CCR7+ and/or CD62L+ naive-like memory regulatory T cells.

3. The composition of any of the preceding claims, not comprising CD4+CD25-CD127high T cells, wherein the composition optionally does not comprise any other cell types.

4. A method for preparing the composition of any of the preceding claims, comprising selecting CD4+CD25+CD127low regulatory T cells, optionally, by sorting cells for their expression of CD4, CD25 and/or CD127.

5. The method of any claim 4, wherein the selection includes positive selection for expression of CD25 and/or depletion of CD127high cells, and/or depletion of CD8+ cells.

6. The method of any of claims 4 or 5, comprising selecting naive regulatory T cells and central memory regulatory T cells and, optionally, naive-like memory regulatory T cells.

7. The method of any of claims 4-5, wherein naive regulatory T cells and central memory regulatory T cells and, optionally, naive-like memory regulatory T cells are positively selected.

8. The method of claim 7, wherein the positive selection includes selection for expression of CCR7.

9. The method of any of claims 7-8, wherein the positive selection includes selection for expression of CD62L.

10. The method of any of claims 4-9, wherein selection comprises
a) contacting cells with antibodies to a cell surface molecule that is to be selected for and
b) cell sorting by a method selected from the group comprising fluorescence-activated cell-sorting (FACS), magnetic-activated cell sorting (MACS) and microfluidics.

11. A method for preparing a regulatory T cell product, comprising carrying out the method of any of claims 4-10 and activating and culturing the composition prepared by said method.

12. The method of any of claims 4-11, further comprising engineering the cells of the composition of any of claims 1-3 or the T cell product of claim 11
a) to express at least one transgene selected from the group comprising a chimeric antigen receptor (CAR) and a transgenic T cell receptor (TCR),
b) to have improved cell survival, and/ or
c) to be resistant to an immunosuppressive drug.

13. The method of any of claims 4-12, further comprising formulating a regulatory T cell product for administration to a subject.

14. A composition or regulatory T cell product obtainable from the method of any of claims 4-13.

15. A pharmaceutical regulatory T cell product comprising the composition of any of claims 1-3 or the regulatory T cell product of claim 14 and optionally, a solvent, preferably, for use in treating a subject having or at risk of having an undesired immune response, such as a subject having an autoimmune disease, an allergy or sepsis or a transplant recipient or a gene therapy recipient.
